# EUROPÄISCHE PATENTSCHRIFT

(11) **EP 2 174 720 B1**
(45) Veröffentlichungstag und Bekanntmachung des Hinweises auf die Patenterteilung: **08.02.2012**
(21) Anmeldenummer: 09012057.7
(22) Anmeldetag: 23.09.2009
(51) Int. Cl.: B05B 11/00, A61M 15/00, B65D 83/14

(54) **Austragvorrichtung mit Sperrmitteln**
Application device with blocking means
Dispositif de distribution avec éléments de blocage

(30) Priorität: 07.10.2008 US 287269
(43) Veröffentlichungstag der Anmeldung: 14.04.2010
(60) Teilanmeldung: 11166779.6
(73) Patentinhaber: Ing. Erich Pfeiffer GmbH, 78315 Radolfzell (DE)
(72) Erfinder: Cater, Miro, Daytona Beach Florida 32124 (US); Körner, Joachim, 88690 Uhldingen-Mühlhofen (DE); Umbeer, Volker, 78315 Radolfzell (DE); Fuchs, Karl-Heinz, 78315 Radolfzell (DE)
(74) Vertreter: Klement, Lukas

(56) Entgegenhaltungen:
- EP-A- 0 472 985
- EP-A2- 1 125 637
- DE-A1- 4 027 391
- DE-A1- 4 133 274
- DE-A1- 19 807 921
- US-A- 4 674 652
- US-A- 4 871 092
- US-A- 5 878 916

## Beschreibung

Die Erfindung betrifft eine Austragvorrichtung für Medien mit einem Gehäuse, einem gegenüber dem Gehäuse manuell bewegbaren Betätigungsglied, das zum Zweck der Betätigung eines Austragmittels aus einer unbetätigten Ausgangslage in Richtung einer Betätigungsrichtung in eine betätigte Endlage überführbar ist, und einem Sperrglied, welches zwischen einer Sperrstellung, in der es die Verlagerung des Betätigungsgliedes in die Endlage verhindert, und einer Freigabestellung, in der es die Verlagerung des Betätigungsgliedes in die Endlage ermöglicht, gegenüber dem Gehäuse verlagerbar ist.

Gattungsgemäße Austragvorrichtungen sind aus dem Stand der Technik bekannt. Sie dienen dem Austrag insbesondere pharmazeutischer Medien wie beispielsweise flüssiger Arzneimittel. Gattungsgemäße Austragvorrichtungen habe ein Gehäuse, in dem ein Austragmittel, beispielsweise eine Kolbenpumpe, vorgesehen ist, das in Reaktion auf eine manuelle Bewegung des Betätigungsgliedes das Medium aus einem Mediumreservoir bis zu einer Austragöffnung der Austragvorrichtung fördert, so dass das Medium an die Umgebung abgegeben wird. Zum Zweck der Betätigung ist üblicherweise eine Fingerauflage vorgesehen, die bestimmungsgemäß von einem Benutzer manuell relativ zum dem Gehäuse niedergedrückt wird, um den Austragvorgang auszulösen. Insbesondere bei Medien, die je nach Anwendung und Dosierung auch eine schädliche Wirkung haben können, ist es zweckmäßig, die Möglichkeit eines Austrags nicht allein in das Ermessen des Bedieners zu stellen. Gattungsgemäße Austragvorrichtungen weisen daher ein verlagerbares Sperrglied auf, welches einen Austragvorgang dadurch unterbinden kann, dass es eine Betätigung des Betätigungsgliedes fallweise mechanisch blockiert. Dieses Sperrglied kann in Abhängigkeit verschiedener Einflussgrößen in seine Freigabestellung verlagert werden, beispielsweise in Reaktion auf eine Authentifizierung des Bedieners mittels eines Zahlencodes oder eines Fingerabdrucks oder in Reaktion auf den Ablauf eines vorgegebenen Zeitintervalls zwischen zwei Austragvorgängen.

Eine gattungsgemäße Austragvorrichtung ist beispielsweise aus der EP 1125637 A2 bekannt. Bei dieser ist im Bereich des Unterbodens das Sperrglied vorgesehen, welches durch einen Elektromagneten zwischen der Freigabestellung und der Sperrstellung hin- und herschaltbar ist.

Aus der WO 2006/095184 A1 ist ebenfalls eine gattungsgemäße Austragvorrichtung bekannt. Bei dieser ist ein Sperrglied vorgesehen, welches radial unter eine Betätigungshandhabe verlagert werden kann, wobei die Verlagerung in diese Sperrstellung durch eine Federkraft erfolgt, während die Überführung in die Freigabestellung über einen Elektromotor realisiert ist.

Ein Spender, der mittels Sperrgliedern eine Betätigung verhindert, ist auch aus der DE 19807921 A1 bekannt. Diese Schrift offenbart hierzu einen Elektromotor, mittels dessen sich kugelförmige Sperralieder radial verlagern lassen.

Aus der EP 472985 A2 ist ein Spender mit einer manuell aufhebbaren Sperre bekannt, die in Reaktion auf eine Betätigung des Spenders aktiviert wird.

Die aus dem Stand der Technik bekannten gattungsgemäßen Austragvorrichtungen weisen eine Reihe von Schwierigkeiten und Nachteilen auf. So sind die Wiederherstellung der Sperrstellung bzw. die Aufrechterhaltung der Sperrstellung bei Ausfall der Stromversorgung, das Problem des hohen Energiebedarfs allgemein sowie die Verhinderung einer Betätigung bei ungewöhnlicher Betätigungsrichtung im Stand der Technik noch nicht vollständig zufriedenstellend gelöst.

### Aufgabe und Lösung

Aufgabe der Erfindung ist es, hinsichtlich der Nachteile des Standes der Technik eine Verbesserung herbeizuführen.

Erfindungsgemäß wird dies dadurch erreicht, dass ein erstes Federmittel vorgesehen ist, durch welches das Sperrglied in Richtung seiner Freigabestellung kraftbeaufschlagt wird und dass das Betätigungsglied und das Sperrglied derart miteinander wirkgekoppelt sind, dass in der Freigabestellung eine Hubbewegung des Betätigungsgliedes aus der unbetätigten Ausgangslage in die betätigte Endlage und/oder eine nachfolgende Rückhubbewegung aus der betätigten Endlage in die Ausgangslage mittels einer Übertragungsmechanik eine Energieeinspeisung in das erste Federmittel bewirkt.

Die erfindungsgemäße Lösung führt dazu, dass sowohl die Energie zur Überführung des Sperrgliedes in die Sperrstellung als auch in die Freigabestellung aus der durch die Betätigung durch den Benutzer aufgebrachte Energie gespeist werden kann. So ist die Energie für die Freigabe im gesperrten Zustand im ersten Federmittel gespeichert. Sobald diese Energie freigegeben wird, bewegt sich das Sperrglied in seine Freigabestellung. Die Rücküberführung des Sperrgliedes wird zumindest abschnittsweise durch die Energie gespeist, die der Benutzer bei der Betätigung in das System einbringt. Vorzugsweise bewirkt die Bewegung des Betätigungsgliedes eine kontinuierliche Verlagerung des Sperrgliedes hin zur Sperrstellung oder darüber hinaus gegen die Kraft des ersten Federmittels und demnach bei gleichzeitigem Spannen des ersten Federmittels. Unter einer kontinuierlichen Verlagerung wird dabei eine Verlagerung verstanden, bei der eine fortschreitende Bewegung des Betätigungsgliedes in vorzugsweise proportionalem Maße gleichzeitig eine Verlagerung des Sperrgliedes bewirkt.

Diese Art der Wirkkopplung erlaubt es, dass Sperrglied durch die vom Bediener aufgebrachte Kraft in Richtung seiner Sperrstellung zu drücken, so dass ohne einen entsprechenden Motor und ohne eine Federkraftbeaufschlagung in Richtung der Sperrstellung alleine durch den Bediener die Sperrstellung wieder hergestellt wird. Die Bewegung des Betätigungsgliedes erfolgt vorzugsweise geradlinig, insbesondere vertikal bezogen auf eine bestimmungsgemäße Ausrichtung der Austragvorrichtung. Die Bewegungsrichtung des Sperrgliedes ist vorzugsweise etwa orthogonal zur Bewegungsrichtung des Betätigungsgliedes ausgerichtet, wobei die Wirkkopplung durch entsprechende Gleitschrägen oder Hebelanordnungen realisierbar ist.

Neben einer unmittelbaren kontinuierlichen Verlagerung des Sperrgliedes während der Betätigung kann die Einspeisung der Energie in das erste Federmittel auch mittelbar erfolgen, beispielsweise unter Nutzung eines weiteren Energiespeichers, der beispielsweise ebenfalls als Federmittel ausgebildet sein kann.

Besonders von Vorteil ist es, wenn sowohl die Hubbewegung als auch die Rückhubbewegung des Betätigungsgliedes mittels einer geeigneten Wirkkopplung genutzt werden, um die Verlagerung des Sperrgliedes zu verursachen. Dabei wird sowohl bei der Hubbewegung als auch bei der Rückhubbewegung eine Energieeinspeisung in das erste Federmittel erzielt. Durch diese Nutzung beider Bewegungen des Betätigungsgliedes relativ zum Gehäuse, ist eine vorteilhafte Übersetzung erzielbar, so dass das gleichzeitig mit der Bewegung des Betätigungsglieds stattfindende Spannen des Federmittels keine nennenswert erhöhte Betätigungskraft seitens des Bedieners erfordert.

Unter einem Federmittel im Sinne der Erfindung wird jedes Bauteil verstanden, welches mittels mechanisch gespeicherter Energie eine Kraftbeaufschlagung verursachen kann. Vorzugsweise handelt es sich um ein Bauteil, in dem die Energie durch elastische Verformung gespeichert ist, insbesondere um eine Schraubenfeder aus Metall oder Kunststoff.

Die durch die Bewegung des Betätigungsgliedes verursachte Bewegung des Sperrgliedes muss das Sperrglied nicht vollständig bis in die Sperrstellung bewegen. Es können auch weitere Mechanismen vorgesehen sein, die über einen Teil der Strecke die Kraftbeaufschlagung des Sperrgliedes in Richtung seiner Sperrstellung bewirken.

Das Betätigungsglied kann einstückig mit einem Abschnitt ausgebildet sein, der bestimmungsgemäß von einem Bediener im Zuge eines Austragvorgangs betätigt wird. Vorzugsweise ist das Betätigungsglied jedoch als separates Bauteil ausgebildet und mit der Betätigungshandhabe zum Zwecke der gemeinsamen Bewegbarkeit verbunden.

Das Austragmittel ist derart ausgebildet, dass durch Hinabdrücken des Betätigungsgliedes zumindest mittelbar ein Austragvorgang verursacht werden kann. Vorzugsweise handelt es sich beim Austragmittel um eine mechanisch mit der Betätigungshandhabe gekoppelte Verdrängerpumpe. Im Sperrzustand der Austragvorrichtung, bei der das Sperrglied in der Sperrstellung angeordnet ist, wird die Bewegungsfreiheit der Betätigungshandhabe oder des mit ihr verbundenen Betätigungsgliedes in einem ausreichenden Maße eingeschränkt, um eine Betätigung des Austragmittels vollständig oder annähernd vollständig zu verhindern. Besonders bevorzugt ist es wenn das Betätigungsglied und das Sperrglied derart miteinander wirkgekoppelt sind, dass infolge der Bewegung des Betätigungsgliedes die Verlagerung des Sperrgliedes vorübergehend über die Sperrstellung hinaus erfolgt. Bei einer solchen Ausgestaltung ist die Wegstrecke, die das Sperrglied in Folge der Hubbewegung und/oder Rückhubbewegung des Betätigungsgliedes gegen die Federkraft des ersten Federmittels zurücklegt, größer als die Wegstrecke des Sperrgliedes zwischen der Sperrstellung und der Freigabestellung. Das Sperrglied wird demzufolge bei der Bewegung in seine Sperrstellung zunächst im Rahmen eines Überhubs über die Sperrstellung hinaus bewegt, um anschließend, beispielsweise bewirkt durch das erste Federmittel, zurück bis in die Sperrstellung gedrückt zu werden. Eine solche Gestaltung kann von Vorteil sein, um die jeweiligen Sperrabschnitte am Sperrglied und am Betätigungsglied, die in der Sperrstellung gemeinsam die Sperrwirkung entfalten, aneinander vorbeizuführen, so dass die Sperrstellung nach einem Austragvorgang zuverlässig wieder erreicht werden kann.

Die Wirkkopplung des Betätigungsgliedes mit dem Sperrglied wird vorzugsweise über eine Kulissenführung erreicht, mittels derer die Hubbewegung und/oder die Rückhubbewegung des Betätigungsgliedes die Verlagerung des Sperrgliedes bewirkt.

Unter einer solchen Kulissenführung wird jeder Mechanismus verstanden, bei dem ortsfest am Sperrglied und ortsfest am Betätigungsglied vorgesehene Abschnitte aneinander abgleiten, während das Betätigungsglied und das Sperrglied sich in unterschiedliche Richtungen zueinander bewegen. Die Bewegungsrichtung des Betätigungsgliedes und des Sperrgliedes schließen vorzugsweise einen Winkel von 90° ein. Vorzugsweise umfasst die Kulissenführung eine Kulissenspur am Sperrglied, die mit einer zum Eingriff vorgesehenen Nocke des Betätigungsgliedes zusammenwirkt. Aber auf die umgekehrte Konstellation ist realisierbar.

Besonders bevorzugt ist es dabei, wenn die Kulissenführung derartig ausgebildet ist, dass sie einen ersten Kulissenspurabschnitt aufweist, in den die Nocke bei der Hubbewegung einfährt, und/oder einen zweiten Kulissenspurabschnitt aufweist, in den die Nocke gegen Ende der Hubbewegung oder beim Übergang in die Rückhubbewegung einfährt.

Es ist dabei ausreichend, wenn einer der beiden genannten Führungsspurabschnitte vorgesehen ist, wobei in jedem Fall die Kulissenführung derartig ausgebildet sein muss, dass die Nocke nach Abschluss der Hubbewegung bei der nachfolgenden Rückhubbewegung einen anderen Bewegungspfad relativ zum Sperrglied beschreibt. Die Kulissenspurabschnitte können als Nut mit beidseitigen Kontaktflächen ausgebildet sein. Es reicht jedoch auch aus, wenn die Kulissenspurabschnitte lediglich einseitig eine Kontaktfläche aufweisen, gegen die die Nocke infolge der Kraft des Federmittels gedrückt wird. Bei einer Gestaltung mit zwei Kulissenspurabschnitten sind diese vorzugsweise in entgegengesetzte Richtung gegen die Betätigungsrichtung des Betätigungsgliedes geneigt, so dass die Hubbewegung und die Rückhubbewegung des Betätigungsgliedes eine gleichgerichtete Verlagerung des Sperrgliedes bewirken.

Um zu gewährleisten, dass die Rückhubbewegung der Nocke bezogen auf das Sperrglied auf einem Bewegungspfad erfolgt, der sich vom Bewegungspfad bei der Hubbewegung unterscheidet, sind vorzugsweise entsprechende Schaltmittel vorgesehen. Diese können dadurch realisiert sein, dass die Nocke im Zuge der Hubbewegung des Betätigungsgliedes quer zur Betätigungsrichtung elastisch ausgelenkt wird, wobei eine Stufe am Sperrglied vorgesehen ist, die im Zuge der Hubbewegung des Betätigungsgliedes bei gleichzeitiger Verminderung der elastischen Auslenkung der Nocke durch die Nocke überschritten wird. Die Stufe ist für die Nocke nur bei der Hubbewegung, nicht aber bei der gegenläufigen Rückhubbewegung überwindbar, so dass nach Überschreiten der Nocke im Zuge der Hubbewegung der Bewegungspfad der Nocke beim Rückhub bezogen auf das Sperrglied vom Bewegungspfad beim Hub unterscheidet. Vorzugsweise ist die Stufe in der Nähe des Umkehrpunktes vorgesehen, also im Bereich der letzten 40%, insbesondere der letzten 25% des Hubweges. Die elastische Auslenkung der Nocke erfolgt vorzugsweise in radialer Richtung und vorzugsweise mittels einer Rampenfläche, deren Fläche mit der Betätigungsrichtung des Betätigungsgliedes eine spitzen Winkel von weniger als 30 ° einschließt. Die elastische Auslenkung erfolgt vorzugsweise unmittelbar durch eine elastische Verformung der Nocke oder eines Stegs, an dem die Nocke vorgesehen ist. Alternativ können jedoch auch separate Federmittel wie beispielsweise eine Metallfeder vorgesehen sein.

Die Stufe stellt vorzugsweise den Übergangspunkt zwischen zwei Kulissenspurabschnitten dar, die in entgegengesetzte Richtung gegenüber der Betätigungsrichtung geneigt sind, um gemeinsam eine gleichgerichtete Bewegung des Sperrgliedes in Folge der Hub- und Rückhubbewegung des Betätigungsgliedes zu bewirken. Es sind jedoch auch Ausgestaltungen mit nur einem Kulissenspurabschnitt in oben beschriebener Art und Weise denkbar, bei denen die Hubbewegung des Betätigungsgliedes keinerlei Bewegung des Sperrgliedes bewirkt und erst der zweite Kulissenspurabschnitt nach Überwindung der Stufe oder einer anderweitigen Umschaltung durch die Schaltmittel gemeinsam mit der Nocke die Bewegung des Sperrgliedes im Zuge der Rückhubbewegung zur Folge hat. Alternativ kann es auch vorgesehen sein, das lediglich die Hubbewegung durch einen ersten Führungsspurabschnitt eine Verlagerung des Sperrgliedes zur Folge hat, während am Ende dieser ersten Führungsspur die Stufe vorgesehen ist, nach deren Überwindung durch die Nocke die Wirkkopplung zwischen Sperrglied und Betätigungsglied entfällt.

Im Sperrzustand wird eine Hubbewegung des Betätigungsgliedes vorzugsweise dadurch zumindest abschnittsweise unterbunden, dass mindestens ein sperrgliedseitiger Sperrabschnitt den Hubbewegungspfad von mindestens einem betätigungsgliedseitigen Sperrabschnitt versperrt, wobei es besonders bevorzugt ist, dass der betätigungsgliedseitige Sperrabschnitt identisch mit der Nocke der Kulissenführung ist. Bei einer solchen Gestaltung hat demnach die Nocke am Betätigungsglied eine Doppelfunktion. In der Sperrstellung des Sperrgliedes bildet sie den Sperrabschnitt, der eine Verlagerung des Betätigungsgliedes und somit einen Austrag unterbindet In der Freigabestellung bildet die Nocke den betätigungsgliedseitigen Teil der Kulissenführung.

Bei einer Weiterbildung der Erfindung eine Steueranordnung vorgesehen, die dafür ausgebildet ist, in einem Blockierzustand die Bewegung des Sperrgliedes gegenüber dem Gehäuse zu blockieren und durch eine mittels eines elektrischen Signals erzielbare Auslösung die Verlagerung des Sperrgliedes aus der Sperrstellung in die Freigabestellung zu ermöglichen.

Eine solche Steueranordnung erlaubt es demnach, das Sperrglied in der Sperrstellung zu halten, bis durch das elektrische Signal, welches beispielsweise der kurzzeitigen Bestromung eines Aktuators dienen kann, dieser Blockierzustand aufgehoben wird. Bei der oben beschriebenen Gestaltung mit einem ersten Federmittel, welches das Sperrglied zumindest mittelbar in Richtung der Freigabestellung kraftbeaufschlagt, führt die Auslösung zur Herstellung des Freigabezustandes.

Das elektrische Signal ist vorzugsweise nicht dafür ausgelegt, die tatsächlich zur Überführung des Sperrgliedes in die Freigabestellung erforderliche Energie zur Verfügung zu stellen, sondern ermöglicht stattdessen die Freigabe zuvor gespeicherter mechanischer Energie zu diesem Zweck.

Besonders bevorzugt ist es, wenn die Steueranordnung derart ausgebildet ist, dass sie bei einer Verlagerung des Sperrgliedes in die Sperrstellung automatisch in den Blockierzustand überführt wird.

Unter einer automatischen Überführung in den Blockierzustand wird in diesem Zusammenhang verstanden, dass ohne Eingreifen einer Elektronik, insbesondere durch einen ausschließlich mechanisch wirkenden Mechanismus die Verlagerung des Sperrgliedes in die Sperrstellung den Blockierzustand zur Folge hat. Dies kann beispielsweise erzielt werden, indem ein in der Sperrstellung wirkendes Rastmittel in Eingriff mit dem Sperrglied kommt. Ebenfalls möglich sind Gestaltungen, bei denen die Blockierung über Magnetkräfte oder Adhäsionskräfte zwischen dem Gehäuse und dem Sperrglied oder einem mit dem Sperrglied verbundenen Sperrhilfsglied genutzt werden.

Die Gestaltung hinsichtlich dieser Weiterbildung führt zu einer sehr einfachen Bauweise, da es nicht erforderlich ist, die Bewegung des Sperrgliedes in die Sperrstellung zu sensieren, um gezielt über die Elektronik der Austragsvorrichtung den Blockierzustand herzustellen.

Bei einer besonders bevorzugten Weiterbildung der Erfindung ist ein Riegelglied vorgesehen, welches im Blockierzustand der Steueranordnung eine Verlagerung des Sperrgliedes oder eines mit dem Sperrglied wirkverbundenen Sperrhilfsgliedes mechanisch blockiert. Im Zusammenhang mit dieser Erfindung wird unter einem Sperrhilfsglied ein Bauteil verstanden, welches derart mit dem Sperrglied verbunden ist, dass es sich bei einer Verlagerung des Sperrgliedes aus der Freigabestellung in die Sperrstellung in eine erste Richtung verlagert und bei einer Verlagerung des Sperrgliedes aus seiner Sperrstellung in die Freigabestellung in eine davon abweichende andere Richtung verlagert. Das Sperrhilfsglied kann derart zwangsgekoppelt sein, dass eine eindeutige Zuordnung zwischen jeder Stellung des Sperrgliedes und einer korrespondierenden Stellung des Sperrhilfsgliedes vorgesehen ist. Das Sperrglied und das Sperrhilfsglied können jedoch auch gedämpft, beispielsweise durch eine Federanordnung, miteinander verbunden werden, so dass trotz der grundsätzlichen Wirkkopplung eine begrenzte Beweglichkeit des Sperrhilfsgliedes auch bei sich nicht bewegendem Sperrglied besteht. Ein besonderer Vorteil bei der Verwendung eines Sperrhilfsgliedes liegt darin, dass die Beweglichkeit des Sperrhilfsgliedes anders geartet sein kann als die Beweglichkeit des Sperrgliedes. So kann insbesondere eine rotative Beweglichkeit des Sperrgliedes vorgesehen sein, wobei das Sperrglied mit einem Sperrhilfsglied wirkgekoppelt ist, welches seinerseits rein translativ beweglich ist.

Die oben beschriebene Weiterbildung mit einem Riegelglied gestattet eine besonders zuverlässige Sicherung des Blockierzustandes und eine einfache Herbeiführung der Auslösung. Als Riegelglied wird dabei ein Bauteil verstanden, welches mechanisch mit dem Sperrglied oder dem Sperrhilfsglied in Eingriff gebracht werden kann, um vorzugsweise formschlüssig die Bewegung des Sperrgliedes beziehungsweise Sperrhilfsgliedes zu unterbinden. Das Riegelglied kann dabei seinerseits selbst in Richtung der blockierenden Eingriffsstellung oder entgegen der Eingriffsstellung durch ein Federmittel kraftbeaufschlagt sein. Zur Herstellung des Blockierzustandes ist das Riegelglied vorzugsweise derart angeordnet und/oder ausgebildet, dass es durch die Überführung des Sperrgliedes in seine Sperrstellung in seinen Eingriffszustand gebracht wird. Dies kann beispielsweise dadurch erreicht werden, dass das Sperrglied oder das Sperrhilfsglied bei der Überführung in die Sperrstellung einen am Riegelglied vorgesehenen Fortsatz erfasst, der das Riegelglied in seiner Gesamtheit verlagert, insbesondere verschwenkt. Auch möglich ist eine Federkraftbeaufschlagung des Riegelgliedes in Richtung des Blockierzustandes. Das Riegelglied kann translativ beweglich sein. Als besonders vorteilhaft wird es jedoch angesehen, wenn das Riegelglied drehbar am Gehäuse gelagert ist.

Bei einer Weiterbildung der Erfindung ist im Blockierzustand der Steueranordnung das Sperrglied oder ein mit dem Sperrglied rückverbundenes Sperrhilfsglied durch einen Permanentmagneten in einer Lage gehalten, aus der resultierend das Sperrglied in seiner Sperrstellung angeordnet ist. Alternativ ist das Riegelglied oder ein mit dem Riegelglied wirkverbundenes Riegelhilfsglied durch einen Permanentmagneten in einer Lage gehalten, aus der die mechanische Blockierung des Sperrgliedes durch das Riegelglied resultiert. Bei diesen beiden Weiterbildungen ist jeweils vorgesehen, dass ein Permanentmagnet zur Herstellung des Blockierzustandes der Steueranordnung vorgesehen ist. Bei einer Steueranordnung ohne Riegelglied ist entweder vorgesehen, dass das Sperrglied selbst oder ein Sperrhilfsglied bei Erreichen der Sperrstellung des Sperrgliedes durch einen Permanentmagneten derart kraftbeaufschlagt wird, dass eine Fixierung des Sperrgliedes bzw. des Sperrhilfsgliedes relativ zum Gehäuse erreicht wird. Bei einer Gestaltung mit einem Riegelglied kann es dieses Riegelglied oder ein damit wirkverbundenes Riegelhilfsglied sein, welches in jener Stellung, in der es die Überführung des Sperrgliedes in die Freigabestellung verhindert, durch den Permanentmagneten relativ zum Gehäuse fixiert wird.

Als Permanentmagnet wird in diesem Zusammenhang sowohl ein permanent magnetisiertes Bauteil verstanden als auch ein permanent stromdurchflossener Leiter, der dadurch ein Magnetfeld erzeugt. Vorzugsweise ist sowohl am entsprechenden beweglichen Glied, also am Sperrglied, dem Sperrhilfsglied, dem Riegelglied oder dem Riegelhilfsglied, als auch am Gehäuse jeweils ein Permanentmagnet vorgesehen. Es kann jedoch auch ausreichen, jeweils nur einen Permanentmagneten und statt des zweiten Permanentmagneten ein magnetisierbares Bauteil vorzusehen. Die im Kontakt dieser Erfindung besonders vorteilhafte Wirkung des Permanentmagneten liegt darin, dass die durch den Permanentmagneten hervorgerufene Kraft, die auf das Sperrglied, Sperrhilfsglied, Riegelglied oder Riegelhilfsglied wirkt, sehr schnell abnimmt, wenn das entsprechende Glied vom gehäusefesten Permanentmagneten entfernt wird, so dass die Wirkung des Permanentmagneten isoliert den Sperrzustand betrifft, während der Überführung in den Freigabezustand jedoch sehr bald vernachlässigbar klein wird.

Bei einer Gestaltung mit einem Permanentmagneten der mittelbar oder unmittelbar dafür verantwortlich ist, dass das Sperrglied in seiner Sperrstellung gehalten wird, ist es besonders bevorzugt, wenn die durch den oder die Permanentmagneten ausgeübte Kraft in einer Endlage, die der Sperrstellung des Sperrgliedes zugeordnet ist, größer ist, als die von Federmitteln der Austragvorrichtung in der Sperrstellung des Sperrgliedes in entgegengesetzte Richtung auf das Sperrglied, das Sperrhilfsglied, das Riegelglied bzw. das Riegelhilfsglied wirkende Kraft ist. Bei einer solchen Gestaltung ist dann, wenn sich das Sperrglied in der Sperrstellung befindet, eine Blockierung dadurch erreichbar, dass die Federkraft, die mittelbar oder unmittelbar das Sperrglied in Richtung der Freigabestellung oder das Riegelglied in Richtung eines entriegelten Zustandes drückt, geringer ist als die in dieser Stellung durch den Permanentmagneten bewirkte Kraft. Das entsprechende Glied wird dementsprechend durch den Permanentmagneten derartig stark kraftbeaufschlagt, dass die in entgegengesetzte Richtung wirkenden Federkräfte keine Bewegung des Sperrgliedes in die Freigabestellung oder des Riegelgliedes in seine entriegelte Stellung bewirken können. Erst wenn das entsprechende Glied durch eine zusätzliche Kraftbeaufschlagung ausreichend weit aus dem Wirkbereich des Permanentmagneten heraus bewegt wird, überwiegt die Federkraft und bewirkt somit unmittelbar oder mittelbar eine Überführung des Sperrgliedes in seine Freigabestellung.

Bei einer Weiterbildung der Erfindung ist zur Erzielung der Auslösung ein elektrisch ansteuerbarer Aktuator vorgesehen, vorzugsweise ein Elektromagnet, durch den entweder das Sperrglied oder ein mit dem Sperrglied wirkverbundenes Sperrhilfsglied kraftbeaufschlagt werden kann, so dass resultierend das Sperrglied in Richtung seiner Freigabestellung verlagert wird. Alternativ kann der elektrische ansteuerbare Aktuator zur Kraftbeaufschlagung des Riegelgliedes oder des mit dem Riegelglied wirkverbundenen Riegelhilfsgliedes Verwendung finden, wobei die Kraftbeaufschlagung in eine Richtung erfolgt, die zu einem Lösen der mechanischen Blockierung des Sperrgliedes durch das Riegelglied und damit zu einer Verlagerung des Sperrgliedes in Richtung seiner Freigabestellung führt. Der Aktuator gemäß dieser Weiterbildung ist dafür vorgesehen, in Reaktion auf eine Bestromung durch ein Steuergerät der Austragvorrichtung eine Kraftbeaufschlagung des entsprechenden Gliedes zu bewirken, um dieses in Richtung der Freigabestellung oder im Falle des Riegelgliedes in Richtung der gelösten Stellung zu verlagern. Dabei diente der Aktuator nicht der Einspeisung der vollständigen hierfür erforderlichen mechanischen Energie, sondern bewirkt lediglich eine Überwindung der zuvor herrschenden Blockierung, beispielsweise indem über einen kurzen Zeitraum, vorzugsweise weniger als 50 ms, eine Kraftbeaufschlagung entgegen der Kraftbeaufschlagung des zuvor beschriebenen Permanentmagneten erfolgt. Das entsprechende Glied, beispielsweise also das Sperrhilfsglied oder das Riegelhilfsglied, wird durch diese kurzzeitige Kraftbeaufschlagung von dem Permanentmagneten gelöst und/oder ausreichend weit aus dessen Wirkbereich entfernt, so dass nachfolgend eine Verlagerung des Riegelglieds in seine gelöste Stellung und/oder des Sperrgliedes in seine Freigabestellung durch die Kraftbeaufschlagung des ersten Federmittels und/oder eines anderen Federmittels erfolgen kann.

Die Verwendung eines Elektromagneten als Aktuator ist hierbei von besonderem Vorteil, da ein solcher Elektromagnet sehr preiswert und klein ausgebildet sein kann. Weiterhin ist die Ansteuerung eines solchen Elektromagneten durch eine Steuerelektronik der Austragvorrichtung denkbar einfach, da eine einfache kurzfristige Bestromung zur Erzielung des gewünschten Effekts ausreicht.

Zur Erreichung des oben beschriebenen Ziels ist es besonders vorteilhaft, wenn der elektrisch ansteuerbare Aktuator dafür ausgebildet ist, eine Kraft auf das Sperrglied, das Sperrhilfsglied, das Riegelglied oder das Riegelhilfsglied auszuüben, die größer ist als die in entgegengesetzte wirkende resultierende Kraft aus der auf das entsprechende Glied wirkenden Kraft des Permanentmagneten und vorzugsweise der Kraft eines das Glied kraftbeaufschlagenden Federmittels.

Durch eine solche Gestaltung, bei der das Steuergerät eine ausreichend starke Bestromung des Aktuator bestimmungsgemäß durchführt, kann eine entsprechende Bewegung des Gliedes entgegen der Kraft des Permanentmagneten erreicht werden, bis die resultierende auf das Glied wirkende Kraft in Richtung der gelösten Stellung bzw. der Freigabestellung größer ist als die in entgegengesetzter Richtung wirkende Kraft des Permanentmagneten.

Bei einer Weiterbildung der Erfindung ist vorgesehen, dass das Riegelglied Teil einer Feldführungseinrichtung ist, die im Blockierzustand der Steueranordnung die Pole des Permanentmagneten miteinander verbinden. Im Blockierzustand ist das Riegelglied gemäß dieser Weiterbildung demnach so angeordnet, dass es mit zwei getrennten Kontaktflächen entweder unmittelbar an den Polen des Permanentmagneten anliegt oder aber mittelbar über an einer der Kontaktflächen oder an beiden Kontaktflächen anliegende weitere Bestandteile der Feldführungseinrichtung mit den Polen gekoppelt ist. Die Bestandteile der Feldführungseinrichtung zeichnen sich durch eine hohe magnetische Permeabilität aus, so dass auch ein vergleichsweise schwacher Permanentmagnet ausreicht, um das Riegelglied im Blockierzustand zuverlässig festzulegen. Die Bestandteile der Feldführungseinrichtung bestehen vorzugsweise aus Metall.

Vorzugsweise ist vorgesehen, dass Sperrglied zur Überführung aus der Sperrstellung in die Freigabestellung um eine in Betätigungsrichtung ausgerichtete Drehachse drehbar ausgebildet ist und über seinen Umfang verteilt mindestens drei Sperrabschnitte aufweist, die in der Sperrstellung des Sperrgliedes derart angeordnet sind, dass eine Hubbewegung des Betätigungsglieds verhindert wird.

Eine solche Ausgestaltung erlaubt eine besonders sichere Sperrstellung, da unabhängig davon, ob und in welche Richtung das Betätigungsglied quer zur Betätigungsrichtung der bestimmungsgemäßen Verwendung niedergedrückt wird, stets mindestens ein Sperrabschnitt vorhanden ist, der ein Bewegen des Betätigungsgliedes mit damit verbundenem Austragvorgang verhindert. Die Drehbeweglichkeit des Sperrgliedes ermöglicht es, diese besonders sichere Sperrstellung bei einer vergleichsweise einfachen mechanischen Gestaltung zu realisieren, da die Drehung eines einstückigen Sperrrings ausreicht, um gleichzeitig alle drei oder mehr Sperrabschnitte in die Sperrstellung zu verlagern. Als besonders bevorzugt wird es angesehen, wenn mindestens sechs Sperrabschnitte am Sperrglied vorgesehen sind und/oder wenn die Sperrabschnitte auf der Kreisbahn gleichmäßig verteilt sind.

Die Drehbeweglichkeit des Sperrgliedes um die Drehachse kann hinsichtlich des Drehwinkels begrenzt sein, so dass eine Überführung aus der Sperrstellung in die Freigabestellung und aus der Freigabestellung in die Sperrstellung in zueinander entgegengesetzten Richtungen durchgeführt wird. Alternativ ist es jedoch in der von Kugelschreibern bekannten Art und Weise auch möglich, die Drehbeweglichkeit des Sperrgliedes nicht zu begrenzen, sondern ein vollständiges Drehen des Sperrgliedes um seiner Drehachse zuzulassen, so dass die Überführung in die Sperrstellung und die Überführung in die Freigabestellung stets in die gleiche Drehrichtung erfolgt, wobei auf Seiten des Betätigungsgliedes und des Sperrgliedes mit jedem Freigabe- und Sperrzyklus andere Sperrabschnitte am Sperrglied und am Betätigungsglied in Kontakt miteinander gelangen, bis das Sperrglied und das Betätigungsglied relativ zueinander eine vollständige 360°-Drehung durchgeführt haben.

Besonders bevorzugt ist es, wenn das Sperrglied das Betätigungsglied umlaufend umgibt und die Drehachse des Sperrgliedes parallel zur Betätigungsrichtung und vorzugsweise koaxial zu einer Mittelachse der Austragvorrichtung ausgerichtet ist. Hierdurch sind besonders kompakte Bauweisen möglich. Das Sperrglied ist bei einer solchen Ausgestaltung in etwa ringförmig ausgebildet und weist eine mittige Ausnehmung auf, durch die hindurch das Betätigungsglied oder eine Betätigungshandhabe mit Austragmitteln der Austragvorrichtung verbunden ist.

Vorzugsweise weisen die sperrgliedseitigen Sperrabschnitte von einer Innenfläche des Sperrgliedes radial nach innen und die korrespondierenden betätigungsgliedseitigen Sperrabschnitte radial nach außen. Eine solche Ausgestaltung ist von Vorteil, da dabei eine Mantelfläche des Sperrgliedes, an deren Innenseite die Sperrabschnitte vorgesehen sind, einen Schutz gegen eine manuelle Beeinflussung durch einen Bediener darstellt. Die Sperrabschnitte, die in der Sperrstellung die Betätigung verhindern, sind durch die Mantelfläche schwer zugänglich und damit gut geschützt.

Bei einer Weiterbildung der Erfindung ist ein Piezomotor oder ein Elektromotor zur Bewegung des Sperrgliedes, des Sperrhilfsgliedes, des Riegelgliedes oder des Riegelhilfsgliedes vorgesehen. Ein solcher Piezomotor ist sehr leicht und hat in Relation zu seinem Bauvolumen eine große Leistung. Der Piezomotor ist vorzugsweise gehäusefest vorgesehen und greift über einen Aktuator am entsprechenden Glied an. Dabei ist die Wirkkopplung zwischen dem Aktuator und dem Sperrglied vorzugsweise dergestalt, dass das Sperrglied gegenüber dem Piezomotor gegen eine Haltekraft, beispielsweise eine Reibungskraft, beweglich ist, da es dies erlaubt, den Piezomotor beispielsweise nur zur Herstellung der Freigabestellung zu nutzen, während die Sperrstellung in der oben beschriebenen Art und Weise mittelbar über die Betätigungshandhabe und ihrer Wirkkopplung mit dem Sperrglied hergestellt wird. Besonders vorteilhaft ist es, wenn die Kopplung zwischen dem Aktuator des Piezomotors und dem Sperrglied lediglich kraftschlüssig ist.

Bei einer Weiterbildung ist der Elektromotor oder der Piezomotor zur Bewegung eines Nockengliedes, vorzugsweise zur rotativen Bewegung eine Nockenscheibe, vorgesehen, wobei das Nockenglied dafür ausgebildet ist, unmittelbar die Bewegung des Sperrgliedes oder eines Sperrhilfsgliedes in der Sperrstellung des Sperrgliedes zu verhindern oder mittelbar über ein Riegelglied die Bewegung des Sperrgliedes oder eines Sperrhilfsgliedes in der Sperrstellung des Sperrgliedes zu verhindern, wobei vorzugsweise das Riegelglied in Richtung einer Auslösestellung Federkraftbeaufschlagt ist und durch das Nockenglied in Abhängigkeit der Stellung des Nockengliedes in der Blockierstellung gehalten wird.

Vorzugsweise ist eine Rückführsperre vorgesehen, die nach Erreichen einer definierten Sperrzwischenstellung bei einer Überführung des Betätigungsgliedes aus der Ausgangslage in die Endlage eine Rückführung des Betätigungsgliedes in die Ausgangslage so lange verhindert, bis das Betätigungsglied in die Endlage überführt wird.

Eine solche Rückführsperre führt dazu, dass nach Beginn einer Betätigung und der daraus resultierenden Überschreitung der Sperrzwischenstellung eine unmittelbare Rücküberführung des Betätigungsgliedes in die Ausgangslage verhindert wird. Diese Rückführung ist erst dann möglich, wenn das Betätigungsglied zuvor bis in die Endlage überführt wurde. Als Endlage ist im Zusammenhang mit dieser Weiterbildung eine Position des Betätigungsgliedes zu verstehen, die nach Entlasten des Betätigungsgliedes zur Herstellung der genannten Sperrstellung führt. Im Falle der Ausgestaltung mit zwei Kulissenspurabschnitten ist die Endstellung beispielsweise erreicht, sobald die Nocke in den zweiten Kulissenspurabschnitt eingerückt wurde.

Diese Gestaltung verhindert einen Missbrauch durch einen Benutzer, der versucht, eine unzulässig hohe Flüssigkeitsmenge auszutragen, indem er ausgehend von der Ausgangslage lediglich einen Teilhub ausführt, um dadurch zu verhindern, dass das Sperrglied aufgrund der Durchführung eines vollständigen Hubes in die Sperrstellung überführt wird. Die Rückführsperre erzwingt, dass nach einer begonnenen Hubbewegung des Betätigungsgliedes dieses zwischenzeitlich auch bis in die Hubendlage überführt wird, um anschließend einen neuen Hub beginnen zu können. Dadurch ist nach Beginn einer Hubbewegung die Ausgangslage nur gemeinsam mit dem Sperrzustand erreichbar.

Eine besondere Ausführungsform der Rückführsperre sieht vor, dass bis zur Erreichung der Endlage keinerlei Rückhubbewegung möglich ist. Dies kann beispielsweise durch eine Rastleiter erreicht werden, die bis zur Erreichung der Endlage stets die fortschreitende Hubbewegung sichert und dadurch auch Teilrückhübe verhindert. Eine einfachere Ausgestaltung sieht vor, dass die Rückführsperre lediglich in einer definierten Sperrzwischenstellung wirkt, so dass nach einer Hubbewegung, die über die Sperrzwischenstellung hinausgeht, ein Rückhub stets nur bis zur Sperrzwischenstellung möglich ist.

Bei einer Weiterbildung dieser Erfindung weist das Austragmittel eine Pumpe mit einer volumenveränderlichen Pumpkammer auf, wobei die Pumpe derart gestaltet ist, dass eine Befüllung der Pumpkammer im Rahmen eines Rückhubs erst ab einer definierten Befüllungszwischenstellung erfolgt, wobei diese Befüllungszwischenstellung derart angeordnet ist, dass sie beim Rückhub erst dann erreicht wird, wenn sich das Betätigungsglied in einer Lage zwischen der Sperrzwischenstellung und der Ausgangslage befindet.

Bei einer solchen Gestaltung ist demnach eine Pumpe vorgesehen, die im Zuge eines Rückhubs nicht kontinuierlich Medium aus einem Mediumreservoir ansaugt, sondern beispielsweise zunächst einen Unterdruck in der Pumpkammer aufbaut, während ein Einlassventil in die Pumpkammer noch geschlossen bleibt. Erst bei Erreichen der Befüllungszwischenstellung öffnet das Einlassventil, so dass durch den aufgebauten Unterdruck schlagartig Medium aus dem Reservoir in die Pumpkammer einströmt. Durch die Anordnung, bei der die Befüllungszwischenstellung der Pumpe erst erreicht werden kann, wenn das Betätigungsglied zwischen der Sperrzwischenstellung und der Ausgangslage angeordnet ist, wird erreicht, dass nach Überschreiten der Sperrzwischenstellung durch das Betätigungsglied in Hubrichtung eine Neubefüllung der Pumpkammer erst möglich ist, wenn zuvor durch Vollendung der Hubbewegung das Betätigungsglied bis in seine Endlage verlagert wurde, da erst die dadurch ermöglichte anschließende Rückhubbewegung in Richtung der Ausgangslage eine Rücküberschreitung der Sperrzwischenstellung in Rückhubrichtung ermöglicht. Damit ist gewährleistet, dass die Neubefüllung der Pumpkammer zwingend mit der Erreichung des Sperrzustandes verbunden ist. Eine missbräuchliche Verwendung durch einen Benutzer, der Teilhubbewegungen zwischen der Sperrzwischenstellung und der Endlage des Betätigungsgliedes durchführt, führt daher nicht zu einem Medienaustrag.

Die Rückführsperre kann bei einer Weiterbildung Rastmittel aufweisen, wobei ein erster Bestandteil der Rastmittel in Betätigungsrichtung ortsfest zum Gehäuse angeordnet ist und wobei ein zweiter Bestandteil der Rastmittel, der zum Zusammenwirken mit dem ersten Bestandteil ausgebildet ist, in Betätigungsrichtung ortsfest zum Betätigungsglied angeordnet ist.

Diese Rastmittel sind derart ausgebildet, dass der zweite Bestandteil der Rastmittel, der ortsfest am Betätigungsglied vorgesehen ist, den ersten Bestandteil nur in Hubrichtung, nicht aber in Rückhubrichtung, überschreiten kann. Bei einer Hubbewegung wird daher der zweite Bestandteil der Rastmittel, beispielsweise ein in radiale Richtung auslenkbares Schnappglied, an dem ersten Bestandteil, beispielsweise einer Schwelle, vorbeigeführt und dabei zwischenzeitlich ausgelenkt. Sobald dieser erste Bestandteil der Rastmittel vom zweiten Bestandteil überschritten wurde, ist eine Rückführung in die Ausgangslage ohne zwischenzeitliches Erreichen der Endlage des Betätigungsgliedes nicht mehr möglich. Die Gestaltung mit Rastmitteln ist einfach in der Herstellung und insbesondere kostengünstig. Als zweiter Bestandteil der Rastmittel können Nocken vorgesehen sein, die mit oben beschriebenen Nocken zum Eingriff in die Kulissenspur identisch sein können oder versetzt zu diesen angeordnet sind. Der erste Bestandteil der Rastmittel, also beispielsweise eine Schwelle, kann im Bereich einer solchen Kulissenspur vorgesehen sein oder getrennt von der Kulissenspur angeordnet sein.

Um zu verhindern, dass die Wirkung der Rückführsperre dadurch umgangen wird, dass mit Gewalt das Betätigungsglied aus einer Teilhubstellung zurück in seine Ausgangsstellung gezogen wird, kann zwischen den von außen zugänglichen Flächen der Austragvorrichtung und dem zweiten Bestandteil der Rastmittel ein Ausgleichsglied vorgesehen sein, welches unter einer derartigen Gewalteinwirkung teleskopisch nachgibt und dadurch die Trennung der Rastmittel verhindert. Denkbar ist auch, dass das Ausgleichsglied als Sollbruchglied vorgesehen ist, welches unter Gewalteinwirkung zerbricht und dadurch dauerhaft weitere Betätigungsvorgänge verhindert.

### Kurzbeschreibung der Zeichnungen

Weitere Vorteile und Merkmale der Erfindung ergeben sich außer aus den Ansprüchen auch aus der nachfolgenden Beschreibung von bevorzugten Ausführungsbeispielen der Erfindung, welche anhand der Darstellungen näher erläutert werden. Dabei zeigen:
- Figuren 1a bis 1f: eine erste Ausführungsform einer erfindungs- gemäßen Austragvorrichtung und
- Figuren 2 bis 5: die Austragvorrichtung der Figuren 1a bis 1f in verschiedenen Stadien der Benutzung, im De- tail:
- Figuren 2a bis 2c: die Austragvorrichtung in einem Sperrzustand,
- Figuren 3a bis 3c: die Austragvorrichtung in einem Freigabezu- stand,
- Figuren 4a bis 4c: die Austragvorrichtung nach erfolgter Hubbewe- gung und
- Figuren 5a bis 5c: die Austragvorrichtung nach erfolgter Rückhub- bewegung, sowie
- Figuren 6a und 6b: eine zweiten Ausführungsform einer erfin- dungsgemäßen Austragvorrichtung, wobei le- diglich die erfindungswesentlichen Komponen- ten dargestellt sind,
- Figuren 7a bis 7d: einzelne Komponenten der Ausführungsform der Figuren 6a, 6b,
- Figuren 8a bis 8c: der Sperrmechanismus der Ausführungsform der Figuren 6a und 6b in verschiedenen Sta- dien der Betätigung,
- Figur 9: eine Ansicht eines Teils einer dritten Ausfüh- rungsform einer erfindungsgemäßen Austrag- vorrichtung,
- Figuren 10a bis 10c: eine Seitenansicht einer vierten Ausführungs- form einer erfindungsgemäßen Austragvorrich- tung,
- Figuren 11a bis 11c: eine fünfte Ausführungsform einer erfindungs- gemäßen Austragvorrichtung in verschiedenen Stadien während der Betätigung,
- Fig. 12a und 12b: das Sperrglied der Ausführungsform gemäß der Fig. 11a bis 11c und

- Fig. 13a bis 13c: eine sechste Ausführungsform einer erfin- dungsgemäßen Austragvorrichtung in verschie- denen Stadien während der Betätigung und
- Fig. 14: eine Steueranordnung der Ausführungsform der Fig. 13a bis 13c in einer Explosionsdarstellung.

### Detaillierte Beschreibung der Ausführungsbeispiele

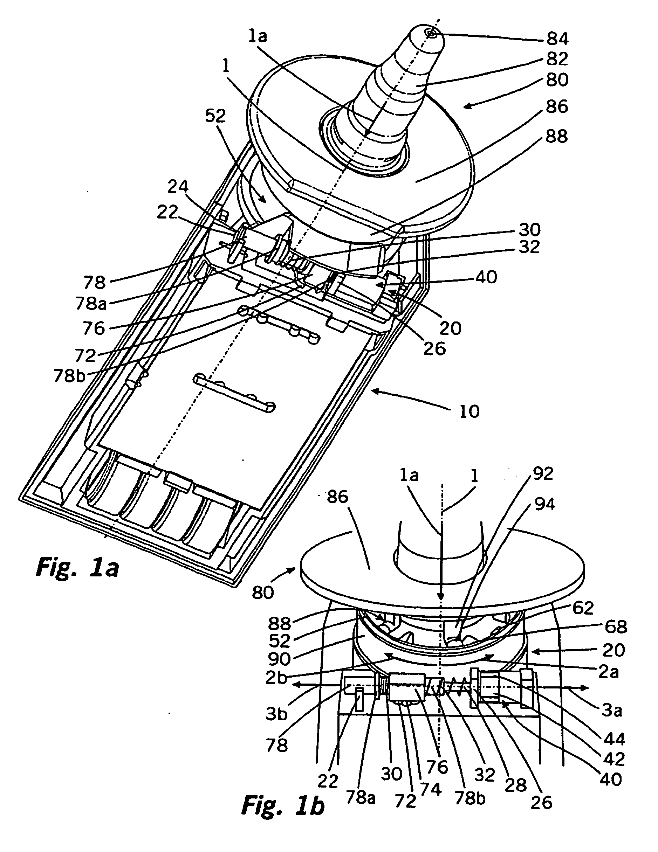

Die Figuren 1a bis 1f zeigen eine erfindungsgemäße Austragvorrichtung bzw. Details davon in verschiedenen Perspektiven.

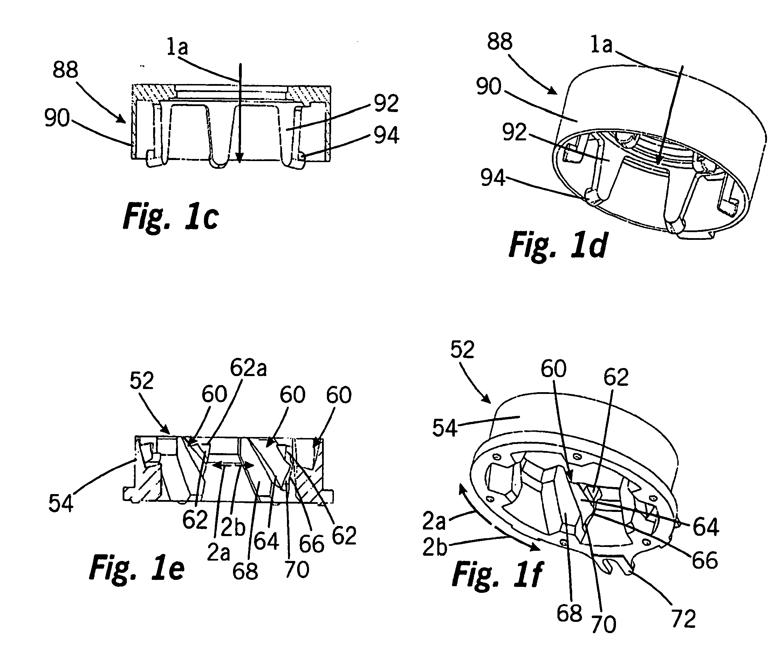

Dabei zeigt die Figur 1a die Gesamtvorrichtung in einem Zustand mit abgenommenem Elektronikmodul, welches im Betrieb frontseitig angekoppelt ist. Die Figur 1b zeigt die Sperrmechanik der Austragvorrichtung im Detail. Die Figuren 1c bis 1f zeigen geschnittene und perspektivische Ansichten eines Betätigungsgliedes und eines Sperrgliedes der Austragvorrichtung, wobei diese Glieder den Kern der Sperrmechanik darstellen.

Die Darstellungen der Figuren 1a bis 1f sollen der Verdeutlichung der einzelnen Elemente der Austragvorrichtung dienen. Das Zusammenwirken wird in den nachfolgenden Figurengruppen 2 bis 5 erläutert.

Die Austragvorrichtung der Fig. 1a weist ein Gehäuse 10 auf, welches neben einem nicht dargestellten Medienreservoir sowie einer nicht dargestellten Pumpeinrichtung eine Sperrmechanik 20 aufweist. Am oberen Ende schließt sich an das Gehäuse 10 oberhalb der Sperrmechanik 20 eine aus mehreren Bestandteilen bestehende, jedoch in sich starre Applikatorbaugruppe 80 an. Diese weist neben einer Nasenolive 82 mit Auslassöffnung 84 eine Betätigungshandhabe 86 auf, die in einer Betätigungsrichtung 1a gegenüber dem Gehäuse 10 translativ gegen die Federkraft einer nicht dargestellten Pumpenfeder beweglich ist. Diese Verlagerung der Applikatorbaugruppe führt in nicht näher dargestellter Art und Weise zu einer Betätigung der im Gehäuse 10 vorgesehenen Pumpe, deren Pumpkammervolumen hierdurch verringert wird, so dass das in der Pumpkammer zuvor vorhandene Medium durch die Auslassöffnung 84 ausgetragen wird.

Die Besonderheit der Austragvorrichtung liegt in dem Sperrmechanismus 20, der dazu dient, in Abhängigkeit von Randparametern die Bewegung der Applikatorbaugruppe 80 in Betätigungsrichtung 1a zu gestatten oder zu unterbinden. Hierfür umfasst der Sperrmechanismus 20 an der Applikatorbaugruppe 80 unterhalb der Betätigungshandhabe 86 ein etwa ringförmiges Betätigungsglied 88. Dieses Betätigungsglied 88 ist in den Figuren 1c, 1d separat abgebildet und in der Fig. 1b zur Verdeutlichung mit teilweise geschnittener Mantelfläche 90 dargestellt.

Innerhalb der Mantelfläche 90 des Betätigungsgliedes 88 erstrecken sich insgesamt sechs Nockenträger 92 vertikal nach unten, an deren Ende jeweils eine nach außen weisende Nocke 94 vorgesehen ist. Die sechs Nockenträger 92 mit Nocken 94 sind auf einem Kreisbogen gleichmäßig angeordnet und somit voneinander jeweils um 60° beabstandet. Das Betätigungsglied 88 und damit auch die Nocken 94 sind fest mit der Betätigungshandhabe 86 und den anderen Elementen der Applikatorbaugruppe 80 verbunden. Ein Niederdrücken der Betätigungshandhabe 88 führt daher stets ebenfalls zu einem Niederdrücken der Nocken 94. Während die Applikatorbaugruppe 80 in der Betätigungsrichtung 1a gegenüber dem Gehäuse 10 beweglich ist, bestehen keine darüber hinausgehenden Freiheitsgrade. So ist insbesondere die Applikatorbaugruppe 80 gegenüber dem Gehäuse 10 nicht um die Achse 1 der Betätigungsrichtung 1a drehbar, sondern verbleibt stets in einer definierten Winkelstellung zum Gehäuse 10.

Korrespondierend zum Betätigungsglied 88 ist ein Sperrglied 52 vorgesehen. Dieses Sperrglied ist separat in den Figuren 1e und 1f dargestellt und auch in der Darstellung der Fig. 1b zu erkennen.

Dieses Sperrglied 52 ist wie auch das Betätigungsglied 88 in etwa ringförmig ausgebildet und wird von einer äußeren Mantelfläche 54 begrenzt, wobei der Außendurchmesser dieser Mantelfläche 54 geringer als der Innendurchmesser der Mantelfläche 90 des Betätigungsgliedes 88 ist, damit die Mantelflächen 54, 90 ineinander geschoben werden können. Anders als das Betätigungsglied 88 ist das Sperrglied 52 in Betätigungsrichtung 1a nicht beweglich, sondern relativ zum Gehäuse 10 stets auf gleicher Höhe angeordnet. Allerdings ist das Sperrglied 52 in begrenztem Umfang um eine Hauptachse 1, die koaxial zur Betätigungsrichtung 1a ausgerichtet ist, in Richtung 2a, 2b drehbar gelagert. Korrespondierend zu den Nocken 94 am Betätigungsglied 88 sind am Sperrglied 52 insgesamt sechs identisch geformte und nach innen weisende Funktionsabschnitte 60 vorgesehen. Diese Funktionsabschnitte 60 erstrecken sich von der Mantelwandung 54 radial nach innen. Sie weisen an ihrem oberen Ende jeweils einen Sperrabschnitt 62 mit einer etwa ebenen oberen Abschlussfläche 62a auf. Darüber hinaus weisen sie jeweils einen ersten Kulissenspurabschnitt 64 sowie einen zweiten Kulissenspurabschnitt 66 auf. Während der erste Kulissenspurabschnitt zum einen vom Sperrabschnitt 62 und zum anderen von einer Erhebung 68 begrenzt wird und in Richtung 1a leicht radial nach innen geneigt ist, wird der zweite Kulissenspurabschnitt 66 lediglich durch die bezogen auf Fig. 1e rechtsseitige Außenkante des Funktionsabschnitts 60 gebildet. Den Übergang vom ersten Kulissenspurabschnitt 64 in den zweiten Kulissenspurabschnitt 66 bildet eine Stufe 70 an dieser rechtsseitigen Außenkante des Funktionsabschnitts 60.

Wie aus den Figuren 1b und 1f deutlich wird, erstreckt sich von der Mantelwandung 54 eine Führungsgabel 72 radial nach außen. Diese führt einen Zapfen 74, welcher einstückig an einem Hülsenelement 76 angebracht ist. Das Hülsenelement 76 ist schwimmend auf einem Stößel 78 gelagert. Im Sinne dieser Erfindung werden das Hülsenelement 76 und insbesondere der Stößel 78 als dem Sperrglied 52 zugeordnete Sperrhilfsglieder verstanden.

Anders als das Sperrglied 52 ist der Stößel 78 nicht um die Drehachse 1 drehbar beweglich, sondern lediglich entlang einer Bewegungsachse 3, die tangential zum drehbaren Sperrglied 52 ausgerichtet ist, translativ in die Richtungen 3a, 3b beweglich.

Der Stößel 78 ist zweiseitig gelagert. Zum einen ist am Gehäuse eine Aufnahme 22 mit einer halbkreisförmigen Ausnehmung 24 vorgesehen, in der das bezogen auf die Figur 1b linksseitige Ende des Stößels 78 liegt. Zum anderen ist rechtsseitig des Stößels 78 eine Anschlagsfläche 26 vorgesehen, von der aus sich ein Führungsdorn 28 in Richtung 3b erstreckt, auf den der Stößel 78 mittels einer nicht dargestellten Bohrung im Stößel 78 aufgeschoben ist.

Linksseitig der schwimmend auf dem Stößel 78 gelagerten Hülse 76 ist ein umlaufender Stößelflansch 78a einstückig am Stößel 78 vorgesehen. Zwischen dem Stößelflansch 78a und der Hülse 26 ist eine als Spiralfeder ausgebildete Stößelfeder 30 vorgesehen. Zwischen dem rechtsseitigen Ende der Hülse 76 und der Anschlagsfläche 26 ist eine Freigabefeder 32 vorgesehen. Das durch die Freigabefeder 32 umgebene rechtsseitige Ende 78b des Stößels 78 ist in nicht näher dargestellter Art und Weise permanentmagnetisch ausgebildet. Zum Zusammenwirken mit diesem permanentmagnetischen Ende 78b des Stößels 78 ist rechtsseitig der Anschlagsfläche 26 gehäusefest eine Magneteinheit 40 vorgesehen, die zum einen einen in Fig. 1b gepunktet dargestellten Permanentmagneten 42 aufweist, der den Stößel in die Richtung 3a kraftbeaufschlagt, und die zum anderen eine ebenfalls gepunktet dargestellten Elektromagneten 44 aufweist, der dafür ausgebildet ist, den Stößel 78 bei Bestromung in Richtung des Pfeils 3b kraftzubeaufschlagen.

Die in den Figuren 1a bis 1f dargestellte Ausführungsform einer erfindungsgemäßen Austragvorrichtung gestattet es mittels des Sperrgliedes und des Betätigungsgliedes, in besonders sicherer und energieeffizienter Art und Weise, wahlweise einen Austragvorgang zu blockieren oder zu ermöglichen.

Die Funktionsweise wird anhand der Figurengruppen 2 bis 5 näher erläutert.

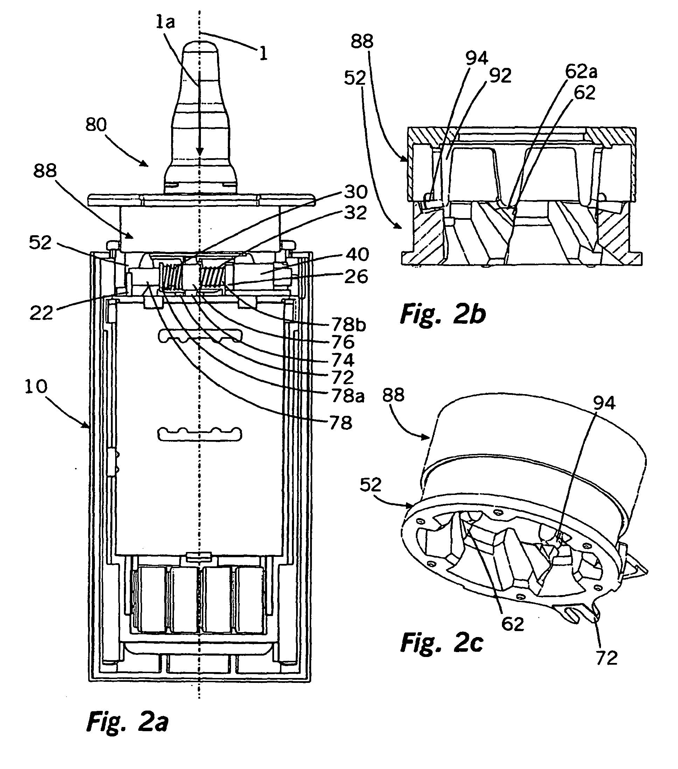

Dabei zeigen die Figuren 2a bis 2c einen Ausgangszustand, in dem ein Austragvorgang blockiert ist. Wie der Figur 2a zu entnehmen ist, liegt in diesem Zustand der Stößel 78 an der Anschlagsfläche 26 an. Trotz der Federkraft der Federn 30,32, die den Stößel 78 in die Richtung 3b kraftbeaufschlagen, löst sich der Stößel 78 von der Anschlagsfläche 26 nicht, da die Permanentmagneten 78b, 42 in der Magneteinheit 40 und am rechtsseitigen Ende des Stößels 78 eine stärkere Kraftbeaufschlagung des Stößels 78 in Richtung 3a bewirken als die Federn 30, 32 in entgegengesetzte Richtung 3b. Die Position des Hülsenelements 76 ergibt sich resultierend aus dem Kräftegleichgewicht an den Federn 30, 32.

Wie aus der zuvor erläuterten Figur 1b deutlich wird, bestimmt die Position des Hülsenelements 76 über den Zapfen 74 und die Führungsgabel 72 zwingend die Drehstellung des Sperrgliedes 52. Aus den Figuren 2b und 2c ist zu entnehmen, welche Drehstellung relativ zu den Nocken 94 beim Zustand der Figur 2a vorliegt. Es ist ersichtlich, dass in dieser Drehstellung die Nocken 94 jeweils direkt oberhalb der Sperrabschnitte 62 angeordnet sind.

Wenn in dem Zustand der Figuren 2a bis 2c eine Betätigung durch den Bediener erfolgt, kann das Betätigungsglied 88 nur in sehr geringem Maße in Richtung 1a verschoben werden. Die Bewegung des Betätigungsgliedes 88 endet, sobald die Nocken 94 auf den obenseitigen Flächen 62a der Sperrabschnitte 62 zum Anliegen kommen. Da das Sperrglied 52 selbst in Richtung 1a nicht beweglich ist, sind ein darüber hinausgehendes Bewegen des Betätigungsgliedes 88 und damit ein Austragen von Medium somit nicht möglich.

Aufgrund der insgesamt sechs Nocken 94, die umlaufend verteilt sind, führt auch eine nicht bestimmungsgemäße, gewalttätige Kraftbeaufschlagung des Betätigungsgliedes 88 orthogonal zur Betätigungsrichtung 1a nicht zu einem Abgleiten aller Nocken 94 von den Flächen 62a der Sperrabschnitte 62. Selbst wenn einseitig ein solches Abgleiten erreicht würde, kann dies niemals für alle Nocken 94 erreicht werden. Der gesperrte Zustand der Austragvorrichtung ist daher vollständig sicher.

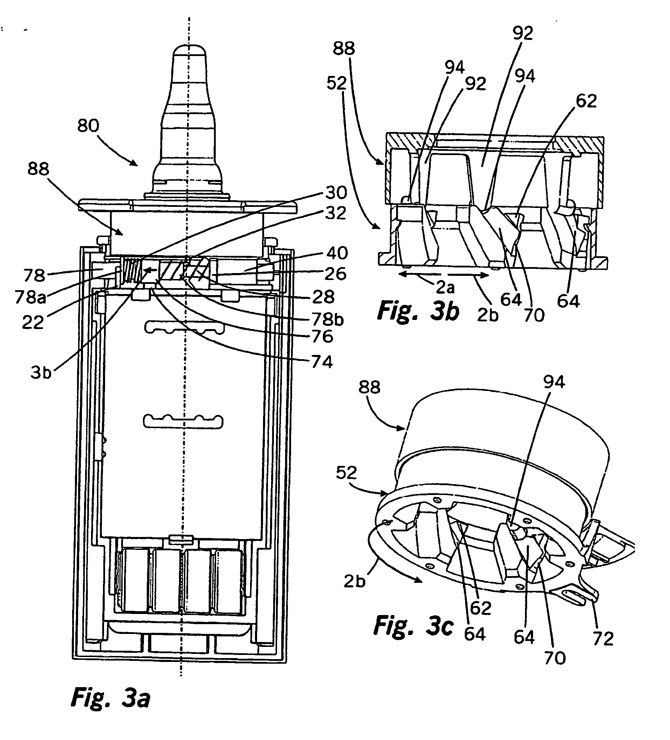

Die Austragvorrichtung ist dafür ausgebildet, dass sie nach einem durch beispielsweise einen Arzt vorgebbaren und in der Elektronik der Austragvorrichtung gespeicherten Zeitintervall nach der vorherigen Benutzung wieder aus ihrer Sperrstellung in ihre Freigabestellung überführt wird. Der Freigabezustand und das Erreichen des Freigabezustands werden nachfolgend anhand der Figuren 3a bis 3c erläutert.

Die Freigabe wird durch Bestromung des Elektromagneten 44 in der Magneteinheit 40 erzielt. Diese Bestromung wird durch ein nicht dargestelltes Steuergerät ausgelöst, welches in Abhängigkeit der genannten Randparameter die Überführung in den Freigabezustand initiiert. Die Bestromung führt zu einer Kraftbeaufschlagung des rechtsseitigen Endes des Stößels 78 in Richtung 3b. Gemeinsam mit der Federkraft der Federn 30, 32 übersteigt die resultierende auf den Stößel 78 in Richtung 3b wirkende Kraft damit die in entgegengesetzte Richtung 3a wirkende Kraft der Permanentmagneten 78b, 42. Demzufolge löst sich der Stößel 78 von der Anschlagsfläche 26 und bewegt sich gespeist von der Federenergie der Feder 30, 32 in Richtung 3b, bis der Stößelflansch 78a an der Aufnahme 22 zum Anschlag kommt.

Die Wirkung der Permanentmagneten 78b, 42 ist örtlich sehr begrenzt, so dass sie schon bei einer geringfügigen Beabstandung des Stößels 78 von der Anschlagsfläche 26 vernachlässigbar klein wird. Während der Bewegung des Stößels 78 muss die Bestromung des Elektromagneten 44 der Magneteinheit 40 daher nicht aufrecht erhalten werden, da bei der entsprechenden Auslegung der Permanentmagneten 78b, 42 und der Federn 30, 32 ein kurzer Impuls ausreicht, um den Stößel 78 bis in die in der Figur 3a dargestellte Stellung zu bringen.

In der dargestellten Endlage des Stößels 78 ergibt sich die Position des Hülsenelements 76 wiederum so, dass die jeweils von den Federn 30 und 32 ausgehende Kraft identisch ist. Aufgrund der Tatsache, dass die rechtsseitige Freigabefeder 32 erheblich härter ist, wird die Hülse 76 vergleichsweise weit in Richtung 3b verschoben. Die Figuren 3b und 3c zeigen die resultierende Relativlage des Betätigungsgliedes 88 und des Sperrgliedes 52. Es ist zu ersehen, dass sich das Sperrglied bezogen auf die Perspektive der Figur 3b in Richtung 2b nach rechts verlagert hat, so dass die Nocken 94 nicht mehr oberhalb der Sperrabschnitte 62 angeordnet sind, sondern oberhalb der Eingangsbereiche der ersten Kulissenspurabschnitte 64. Demzufolge entfällt in dieser Freigabestellung die Limitierung der Bewegungsfreiheit der Nocken 94 nach unten.

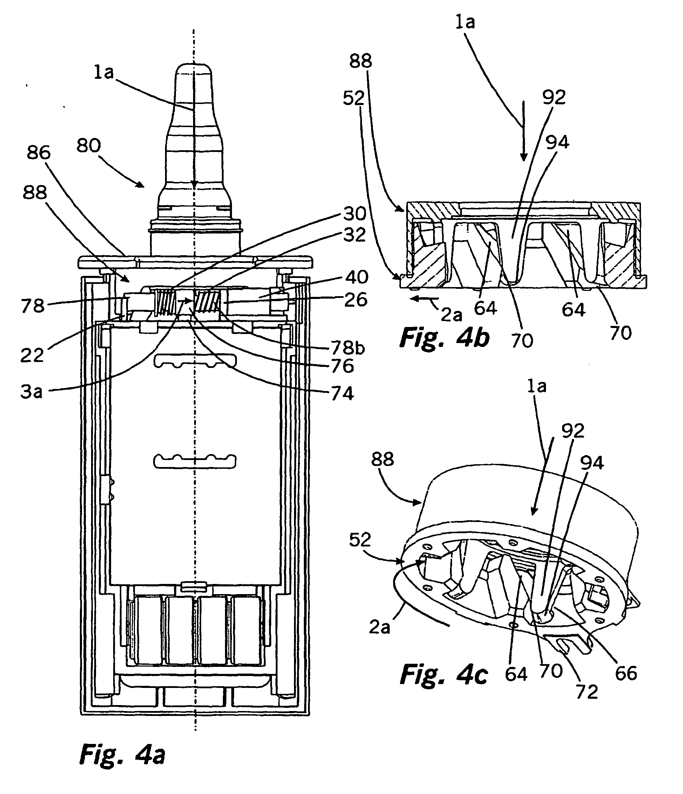

Ausgehend von diesem Freigabezustand der Figuren 3a bis 3c kann daher nun eine Hubbewegung zur Erzeugung eines Medienaustrags erfolgen. Bei der Bewegung des Betätigungsgliedes 88 in Richtung 1a nach unten fahren die Nocken 94 in die ersten Kulissenspurabschnitte 64 ein und verdrehen in Folge der Schrägstellung dieser Kulissenspurabschnitte 64 das Sperrglied 52 bezogen auf die Perspektive der Figur 3b nach links in Richtung 2a. Das Hinabdrücken des Betätigungsgliedes 88, welches mit einem Austragvorgang einhergeht, geht demnach gleichzeitig mit einem Bewegen des Sperrgliedes 52 in Richtung der Sperrstellung einher. Die L-förmige Gestalt der Nockenträger 92 und der Nocken 94 sorgt dafür, dass die Nockenträger 92 während der Bewegung vom Zustand der Fig. 3a in den Zustand der Fig. 4a nicht mit dem Sperrabschnitt 60 kollidieren.

Während der Bewegung des Betätigungsgliedes 88 nach unten in Richtung 1a wird ein elastischer Spannungszustand in den Nockenträgern 92 erzeugt, da die Nocken 94 aufgrund der Formgebung der ersten Führungsspurabschnitte 64 radial zunehmend nach innen ausgelenkt werden. Sobald die Nocken 94 das untere Ende der Führungsspurabschnitte 64 erreicht haben, werden sie über die das Ende bildende Stufe 70 hinübergeschoben, was eine schlagartige Entspannung der gespannten Nockenträger 92 zur Folge hat. Dabei verlagern sich die Nocken 94 wieder radial nach außen.

Die Figuren 4a bis 4c zeigen den dadurch erreichten Zustand. In diesem Zustand, der in etwa den Umkehrpunkt zwischen Hubbewegung und Rückhubbewegung darstellt, ist aufgrund der bis dahin erfolgten Drehbewegung des Sperrgliedes 52 bereits wieder der Zustand erreicht, in dem der Stößel 78 an der Anschlagsfläche 26 anliegt. Er ist daher in der oben beschriebenen Art und Weise in diesem Zustand bereits wieder durch die Permanentmagneten 78b und 42 gehalten.

Der Austragvorgang ist beim Erreichen des Zustandes der Figuren 4a bis 4c bereits abgeschlossen. Ausgehend vom Zustand der Figuren 4a bis 4c erfolgt die Rückhubbewegung, sobald der Bediener die Kraftbeaufschlagung des Betätigungsgliedes 88 nach unten in Richtung 1a entfallen lässt. Sobald dies geschieht, wird die gesamte Applikatorbaugruppe 80 mitsamt dem Betätigungsglied 88 durch die nicht dargestellte Rückstellfeder der Pumpeinrichtung entgegen der Richtung 1a nach oben verlagert. Aufgrund der Tatsache, dass die Nocken 94 über die Stufe 70 hinausbewegt wurden, ist ein entgegengesetzter Bewegungspfad der Nocken 94 entlang der ersten Kulissenspurabschnitte 64 nicht möglich. Stattdessen bewegen sich die Nocken 94 entlang der zweiten Kulissenspurabschnitte 66, also entlang der rechtsseitigen Kante der Funktionsabschnitte 60 nach oben, so dass die Verdrehung des Sperrgliedes 52 bezogen auf die Figuren 4b nach links in Richtung 2a noch fortgesetzt wird. Da eine korrespondierende Bewegung des Stößels 78 in Richtung 3a nicht mehr möglich ist, da der Stößel 78 in dieser Phase bereits an der Anschlagsfläche 26 anliegt, wird durch dieses Weiterdrehen des Sperrgliedes 52 in Richtung 2a lediglich noch das Hülsenelement 76 mitverlagert. Gleichzeitig wird die rechtsseitige Freigabefeder 32 zwischen dem Hülsenelement 76 und der Anschlagsfläche 26 noch weiter gestaucht.

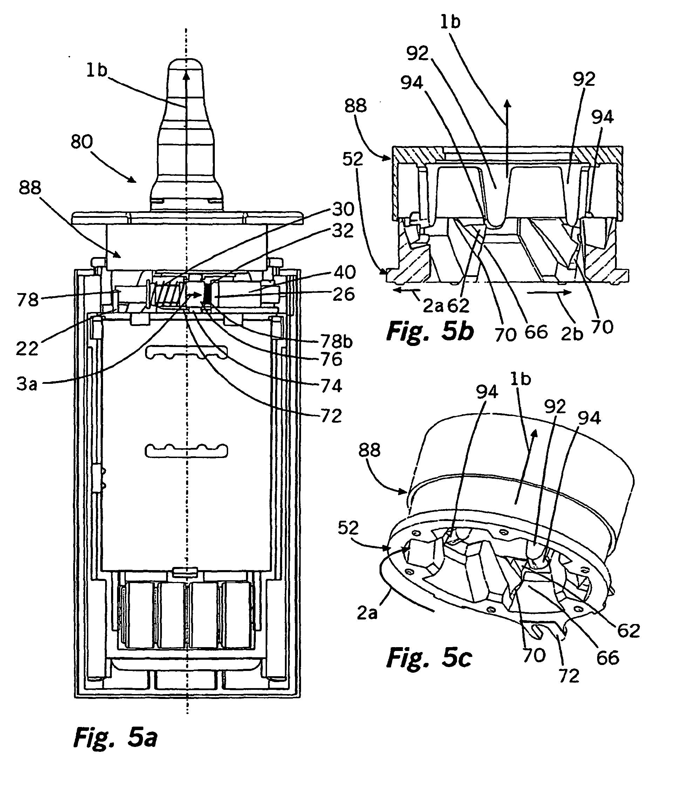

Die Figuren 5a bis 5c zeigen die letzte Phase des Rückhubs. Wie anhand der Figur 5a zu ersehen ist, erreicht die Freigabefeder 32 zwischenzeitlich einen extrem gestauchten Zustand, der erreicht wird, wenn, wie in den Figuren 5b und 5c ersichtlich, die Nocken 94 im Bereich der rechtsseitigen Spitze der Sperrabschnitte 62 an der Außenkante der Funktionsabschnitte entlanggleiten.

In dem Augenblick, in dem die Nocken 94 außer Eingriff mit den Sperrabschnitten 62 gelangen und damit den zweiten Führungsspurabschnitt 66 verlassen, drückt die extrem gestauchte Freigabefeder 32 die Hülse schlagartig nach links, was in der Perspektive der Figuren 5b und 5c ein Verdrehen des Sperrgliedes 52 nach rechts entgegen der Richtung 2a zur Folge hat. Diese Bewegung endet, sobald an den Federn 30, 32 wieder ein Kräftegleichgewicht herrscht. Durch die Bewegung werden die Nocken 94 relativ zu den Sperrabschnitten 62 wieder so verlagert, dass sie ihre Sperrlage oberhalb der Sperrabschnitte 62 einnehmen. Damit ist wieder der Zustand der Figuren 2a bis 2c erreicht.

Die beschriebene Gestaltung ermöglicht einen Sperrmechanismus für eine Austragvorrichtung, bei dem für die Bewegung des entsprechenden Sperrgliedes keinerlei Energiequelle in der Austragvorrichtung vorgesehen sein muss, da die Überführung in die Sperrstellung unmittelbar durch die vom Bediener eingebrachte Energie gespeist wird und da die Überführung in die Freigabestellung mittelbar durch die vom Bediener eingebrachte Energie, zwischengespeichert in den Federn 30, 32, gespeist wird. Nur zum Auslösen der Bewegung in die Freigabestellung ist ein Auslösemittel, beispielsweise in Form eines Elektromagneten 44, vorgesehen. Die Aufgabe dieses Elektromagneten 44 ist jedoch nicht die Aufbringung der zur Verlagerung des Sperrgliedes erforderlichen Energie, sondern die lediglich kurzzeitige Bewirkung einer geringen zusätzlichen Kraft auf den Stößel, die gemeinsam mit der Federkraft der Federn 30, 32 ein Ablösen des Stößels von der Anschlagsfläche 26 bewirkt.

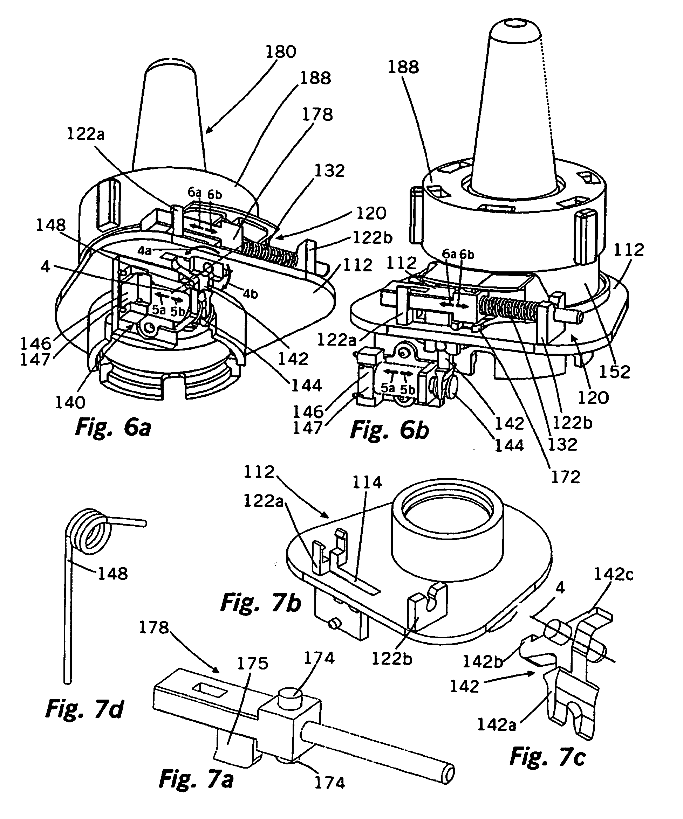

Die Figuren 6a und 6b zeigen eine zweite Ausführungsform einer erfindungsgemäßen Austragvorrichtung. Diese stimmt bezüglich vieler Merkmale und Komponenten mit der ersten Ausführungsform überein. Insbesondere sind das Betätigungsglied 188 sowie das Sperrglied 152 nahezu identisch mit der vorbeschriebenen Ausführungsform gestaltet.

Unterschiede sind insbesondere im Hinblick auf die Sperrmechanik 120 vorgesehen. Diese Unterschiede werden anhand der Figuren 6a und 6b sowie der Einzelteildarstellungen der Figuren 7a bis 7d nachfolgend näher erläutert.

Wie insbesondere der Figur 6b zu entnehmen ist weist das drehbar gelagerte Sperrglied 152 auch bei dieser zweiten Ausführungsform radial nach außen weisende Führungsgabeln 172 auf, mittels derer eine Kopplung zwischen dem Sperrglied 154 mit translativ beweglichen Teilen des Sperrmechanismus 120 realisiert ist. Abweichend von der Ausführungsform der vorangegangenen Figuren ist in die Führungsgabeln 172 bei der zweiten Ausführungsform jedoch ein in sich starres Stößelbauteil 178 als Sperrhilfsglied eingesetzt, welches detailliert in der Figur 7a dargestellt ist. Dieses Stößelbauteil 178 ist über zwei Zapfen 174 in die Führungsgabeln 172 eingesetzt und darüber hinaus gehäuseseitig in zwei Lagerschalen 122a, 122b gelagert, so dass das Stößelbauteil 178 lediglich translativ beweglich ist. An der Unterseite des Stößelabschnitts 178 ist eine Sperrnocke 175 vorgesehen, die durch einen Spalt 114 in einer obenseitig das Gehäuse 110 abschließenden und in Fig. 7b näher dargestellten Platte 112 hindurchgreift, um mit im Weiteren noch beschriebenen anderen Komponenten der Sperrmechanik 120 zusammenzuwirken. Durch eine Freigabefeder 132 wird das Stößelbauteil 178 permanent nach links in Richtung 6a, also in Richtung der Freigabestellung, kraftbeaufschlagt.

Wie sich aus der Zwangskopplung des Stößelbauteils 178 und des Sperrgliedes 152 ergibt, kann über die Lage des Stößelbauteils 178 gesteuert werden, ob die Austragvorrichtung sich in einem Freigabezustand oder einem Sperrzustand befindet. Zur Erreichung des Freigabezustandes muss das Stößelbauteil 178 in seine linksseitige Endlage verschoben werden, während es in seiner rechtsseitigen Endlage eine Betätigung der Austragvorrichtung behindert.

Unterhalb der Platte 112 ist eine Steueranordnung 140 vorgesehen, die mit der Sperrnocke 175 bestimmungsgemäß zusammenwirkt. Diese Steueranordnung 140 umfasst einen schwenkbaren Riegel 142, der um eine Drehachse 4 an der Gehäuseplatte 112 angelenkt ist. Wie in Fig. 7c dargestellt ist, weist der Riegel 142 einen nach unten weisenden Betätigungsabschnitt 142a, einen bezogen auf die Darstellung der Figuren 6a, 6b nach links weisenden Eingriffsabschnitt 142b und einen nach rechts weisenden Rückstellabschnitt 142c auf. Wie insbesondere die Figuren 6a und 6b zeigen ist der Riegel 142 über den Betätigungsabschnitt 142a mit einem translativ entlang der Achse 5 beweglichen Riegelhilfsglied 144 derart gekoppelt, dass eine translative Bewegung dieses Regelhilfsglieds 144 eine Schwenkbewegung des Riegels 142 zur Folge hat. Zur Kraftbeaufschlagung des Riegelhilfsgliedes 144 sind in nicht näher dargestellter Art und Weise ein Permanentmagnet 146 und ein Elektromagnet 147 vorgesehen, wobei der Permanentmagnet 146 dafür ausgebildet ist, das Riegelhilfsglied 144 in Richtung des Pfeils 5a kraftzubeaufschlagen, und der Elektromagnet 147 dafür ausgebildet ist, bei Bestromung das Riegelhilfsglied 144 in Richtung des Pfeils 5b kraftzubeaufschlagen.

Der Eingriffsabschnitt 142b des Riegels 142 dient zum Zusammenwirken mit der Sperrnocke 175. In dem Sperrzustand der Austragvorrichtung, der in den Figuren 6a und 6b dargestellt ist, blockiert der Eingriffsabschnitt 142b eine Verlagerung der Stößelbaugruppe 178 in Richtung des Pfeils 6a nach links. Erst wenn der Riegel 142 um die Drehachse 4 in Richtung des Pfeils 4a verschwenkt wird, entfällt die Blockierung des Stößelbauteils 178, so dass die Freigabestellung des Stößelabschnitts 178 erreichbar ist. Der Riegel 142 wird durch die Schenkelfeder 148, die in Fig. 7d dargestellt ist, permanent in Richtung des Pfeils 4a momentenbeaufschlagt, wobei dies in der Stellung der Figuren 6a und 6b nicht reicht, um den Riegel 142 zu verschwenken, da dieser durch das Riegelhilfsglied 144 in seiner in den Figuren 6a und 6b dargestellten Positionen gehalten wird.

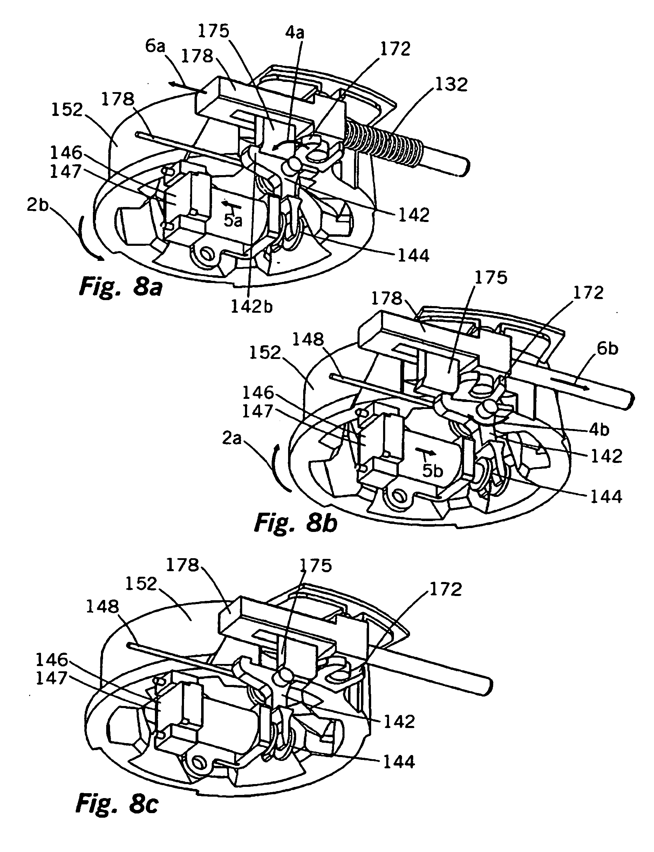

Anhand der Figuren 8a bis 8c soll nachfolgend die Funktionsweise im Detail erläutert werden.

Die Figur 8a zeigt den gesperrten Ausgangszustand der Austragvorrichtung, der auch in den Figuren 6a und 6b dargestellt ist. Wie bereits erläutert, ist in dieser Ausgangsstellung des Stößelbauteils 178 in Richtung 6a nicht möglich, da die Steuernocke 175 durch den Eingriffsabschnitt 142b in ihrer Bewegungsfreiheit in Richtung 6a begrenzt ist. In diesem Sperrzustand 142 ist der Riegel 142 zwar durch die Feder 148 in Richtung des Pfeils 4a momentenbeaufschlagt, diesen Momentenbeaufschlagung reicht jedoch nicht aus, um den Riegel 142 zu drehen, da im dargestellten Zustand das Regelhilfsglied 144 in nicht näher dargestellter Art und Weise am Permanentmagneten 146 anliegt und das dadurch erzeugte Haltemoment auf dem Riegel 142 größer ist als das durch die Feder 148 erzeugte Moment.

Im Sperrzustand der Figur 8a ist eine Betätigung der Austragvorrichtung aus den gleichen Gründen wie bei der zuvor beschriebenen ersten Ausführungsform der Fig. 1 bis 5 nicht möglich. Bei einem Herabdrücken des Betätigungsgliedes 188 würden die im Zusammenhang mit dieser zweiten Ausführungsform nicht dargestellten Nocken des Betätigungsgliedes 188 durch die Sperrabschnitte des Sperrgliedes 152 an einer für einen Austragvorgang erforderlichen Verlagerung gehindert.

Erst wenn durch die nicht dargestellte Steuerelektronik eine Bestromung des Elektromagneten 147 stattfindet, kann hierdurch der Freigabezustand der Austragvorrichtung herbeigeführt werden. Die Bestromung des Elektromagneten 147 führt zu einer kurzzeitigen Kraftbeaufschlagung des Riegelhilfsgliedes 144 in Richtung 5b nach rechts. Dabei braucht die Kraftbeaufschlagung durch den Elektromagneten 147 nicht sehr stark zu sein, da sie lediglich mit der bereits am Regelhilfsglied 144 anliegenden Kraftbeaufschlagung durch die Schenkelfeder 148 zusammen die durch den Permanentmagneten 146 in entgegengesetzte Richtung wirkende Haltekraft überwinden muss. Die aus der Bestromung des Elektromagneten 147 resultierende Verlagerung des Riegelhilfsgliedes 144 in Richtung 5b führt aufgrund der Wirkkopplung des Riegelhilfsglieds 144 mit dem Riegel 142 gleichzeitig zu einem Verschwenken des Riegels 142 in Richtung des Pfeils 4a. Dadurch wird der Eingriff zwischen dem Eingriffsabschnitt 142b und der Sperrnocke 175 gelöst, so dass das Stößelbauteil 178 in Richtung 6a nach links verlagert wird, bis die Sperrnocke in nicht näher dargestellter Art und Weise am Ende der Ausnehmung 114 anschlägt. Die erforderliche Energie zur Verlagerung des Stößelbauteils 178 stammt dabei von der zum Zwecke der Vereinfachung lediglich in der Figur 8a dargestellten Freigabefeder 132.

Der Zustand der Figur 8b stellt den hierdurch erreichten Freigabezustand der Austragvorrichtung dar. Durch die Verlagerung der Stößelbaugruppe 178 hat ebenfalls eine Verdrehung des Sperrgliedes 152 in Richtung 2b stattgefunden, so dass das Sperrglied 152 nunmehr in einer Position ist, in der die Nocken des Betätigungsgliedes 188 an den Sperrabschnitten des Sperrgliedes 152 vorbei bewegt werden können, so dass ein Austragvorgang möglich ist. Dies entspricht dem Zustand der Fig. 3a bis 3c der ersten Ausführungsform.

Wie es bereits zur ersten Ausführungsform beschrieben wurde, führt der Austragvorgang im Rahmen der Hub- und der Rückhubbewegung wiederum zu einem Verdrehen des Sperrgliedes 152 in Richtung des Pfeils 2a. Aufgrund der Zwangskopplung des Sperrgliedes 152 mit dem Stößelbauteil 178 wird dadurch auch das Stößelbauteil 178 in Richtung des Pfeils 6b bewegt. Diese Bewegung hat in einem ersten Teilabschnitt zunächst keine Konsequenzen auf die Position des Riegels 142. Erst wenn die Sperrnocke 175 in den Bereich des Rückholabschnitts 142c des Riegels 142 gelangt wird der Riegel 142 in Richtung 4b gegen die Kraft der Schenkelfeder 148 zurückgeschwenkt, bis das Riegelhilfsglied 144 wieder in eine Lage kommt, in der es durch den Permanentmagneten 146 gehalten wird. Der dadurch erreichte Zustand ist in der Fig. 8c dargestellt. Hinsichtlich des Sperrgliedes 152 und des Betätigungsgliedes 188 ist dieser Zustand mit dem der Fig. 5a bis 5c vergleichbar.

In gleicher Art und Weise wie es bereits im Zusammenhang mit der ersten Ausführungsform beschrieben wurde, erfolgt die Bewegung des Sperrgliedes 152 im Zuge der Hub- und Rückhubbewegung des Betätigungsgliedes 188 über die Sperrstellung des Stößelbauteils 178 nach rechts in Richtung 6b hinaus, wobei dann, wenn die Nocken des Betätigungsgliedes 188 außer Eingriff mit den Sperrabschnitten des Sperrgliedes gelangen abschließend eine in entgegengesetzte Richtung 6a erfolgende Bewegung des Stößelbauteils 178 das Stößelbauteil 178 wieder in die Sperrstellung der Fig. 6a, 6b und 8a bringt. In dieser Sperrstellung liegt die Sperrnocke 175 wieder am Eingriffsabschnitt 142b des Riegelgliedes 142 an, so dass ein Austragvorgang bis zur nächsten Auslösung durch Bestromung des Elektromagneten 147 unterbunden wird.

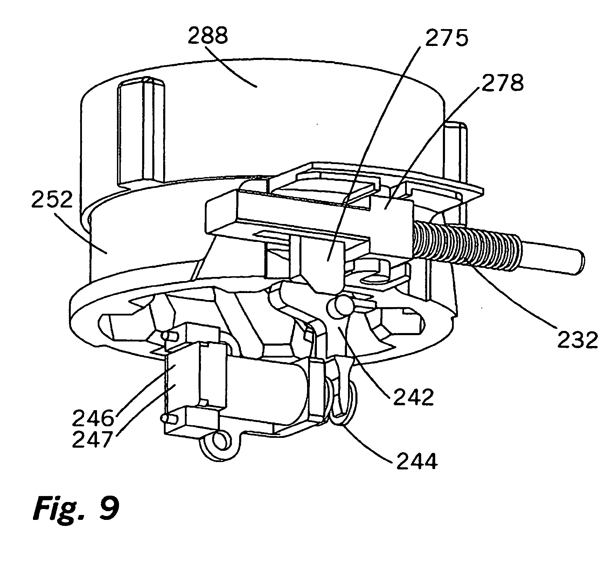

Bei einer in Fig. 9 dargestellten Variation zu dieser zweiten Ausführungsform entfällt die Schenkelfeder 148, die das Riegelglied 142 in Richtung 4a momentenbeaufschlagt. Stattdessen erfolgt die Momentenbeaufschlagung des Riegelgliedes 242 in die Stellung, in der es die Sperrnocke 275 nicht mehr behindert, durch die Sperrnocke 275 und die Freigabefeder 232 selbst. Zu diesem Zweck ist der Kontaktbereich der Sperrnocke 275 so geformt, dass der Kontaktbereich in der dargestellten Sperrstellung das Riegelglied 242 permanent momentenbeaufschlagt. Dies führt zusammen mit der gleichgerichteten Momentenbeaufschlagung durch den Elektromagneten 247 zur Überwindung der Kraft des Permanentmagneten 246 und zum Verschwenken des Riegelgliedes 242, sobald der Elektromagnet 247 bestromt wird.

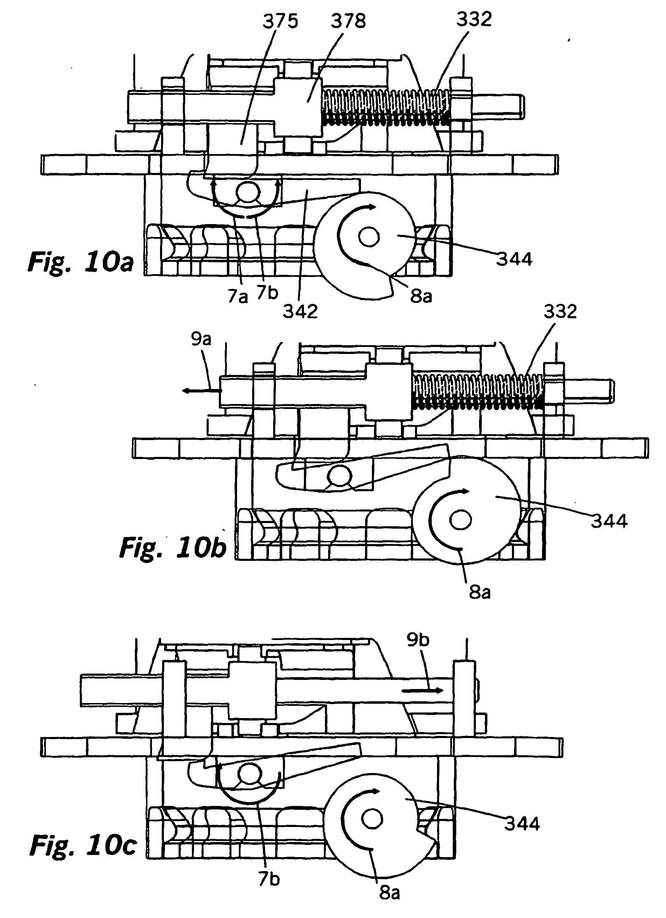

Die Figuren 10a bis 10c zeigen eine weitere Ausführungsform einer erfindungsgemäßen Austragvorrichtung in einer Seitenansicht. Der Unterschied zu der zweiten Ausführungsform liegt dabei darin, dass der Riegel 342 in Richtung seiner Blockierstellung, also in Richtung des Pfeils 7a durch eine nicht dargestellte Feder momentenbeaufschlagt wird und dadurch die Sperrnocke 375 im Sperrzustand der Fig. 10 blockiert.

Um eine Freigabe der Sperrnocke 375 zu erreichen wird der Riegel 342 mittels einer Nockenscheibe 344 verschwenkt. Dabei wird durch eine Drehung dieser Nockenscheibe 344 in Richtung des Pfeils 8a der Riegel 342 demnach soweit in Richtung des Pfeils 7b verschwenkt, dass die Sperrnocke 375 und damit das Stößelbauteil 378 außer Eingriff geraten und unter der Wirkung der Freigabefeder 332 in Richtung 9a nach links verschoben werden können. Der Zustand unmittelbar vor der Auslösung ist in der Figur 10b dargestellt. Durch die Auslösung wird der Freigabezustand erreicht, der in Fig. 10c dargestellt ist.

Nach dieser Auslösung wird die Nockenscheibe 344 in Richtung des Pfeils 8a weitergedreht, bis sie wieder ihre Grundstellung der Figur 10a einnimmt. Sobald ausgehend vom Freigabezustand der Figur 10c durch eine Austragbetätigung das Stößelbauteil 378 wieder in Richtung 9b nach rechts verschoben wird, gelangt der Riegel 342 aufgrund seiner Federmomentenbeaufschlagung in Richtung 7a wieder in Eingriff mit der Sperrnocke 375, so dass sich der Sperrzustand der Figur 9a wieder einstellt.

Anders als bei den vorangegangenen Ausführungsformen erfolgt bei dieser Ausführungsform das Auslösen und damit die Herstellung des Freigabezustandes nicht über einen Elektromagneten, sondern über einen beliebig gearteten Motor, der zur Rotation der Nockenscheibe 344 vorgesehen ist. Bei einem solchen Motor kann es sich beispielsweise um einen kleinen Elektromotor oder vorzugsweise um einen Piezomotor handeln. Wie auch bei den vorangegangenen Ausführungsformen dient dieser Motor nicht der Zurverfügungstellung der mechanischen Energie, um das Sperrglied 352 in seine Freigabestellung oder seine Sperrstellung zu bewegen, sondern lediglich der Auslösung zur Überführung des Sperrgliedes 352 in seine Freigabestellung, so dass der genutzte Motor keine hohe Motorleistung erreichen muss.

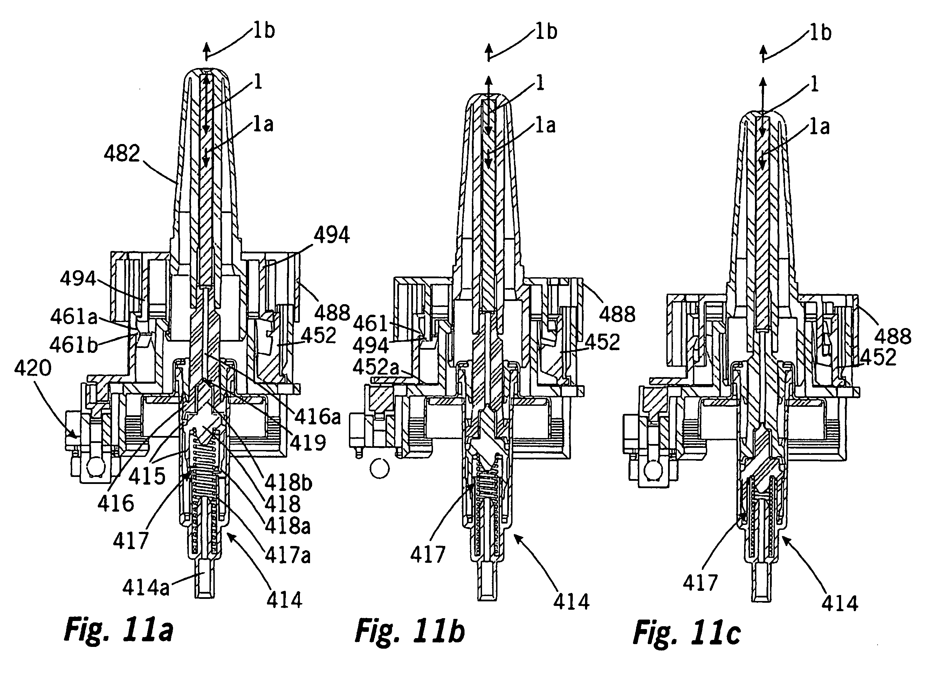

Die Fig. 11a bis 12b zeigen eine weitere Ausführungsform einer erfindungsgemäßen Austragvorrichtung. Dabei zeigen die Fig. 11a bis 11c Teilansichten der Austragvorrichtung, die hinsichtlich der meisten Bestandteile mit der Ausführungsform der Fig. 1 bis 5 übereinstimmt. Die Austragvorrichtung weist eine Sperrmechanik 420 auf, die mit der Sperrmechanik 20 der Fig. 1 bis 5 übereinstimmen kann. Gleichermaßen könnte jedoch auch die Sperrmechanik 120 der Fig. 6 bis 8 oder die Sperrmechanik der Fig. 9 und 10 Verwendung finden.

Dargestellt ist auch eine innerhalb des Gehäuses der Austragvorrichtung angeordnete Pumpe 414 mit einer Pumpkammer 415. Bei dieser Pumpe 414 handelt es sich um eine Kolbenpumpe, deren Kolben 416 zum Zwecke der Volumenveränderung der Pumpkammer 415 gemeinsam mit dem Betätigungsglied 488, der Nasenolive 482 und einer nicht dargestellten Betätigungshandhabe in Richtung des Pfeils 1 manuell verlagerbar ist. Die Besonderheit dieser Pumpe liegt in der Gestaltung des Einlassventils 417. Dieses Einlassventil 417 umfasst eine an einem Ventilkörper 418 vorgesehene umlaufende und nach innen gewandte Ventillippe 418a am unteren Ende des Ventilkörpers 418 und korrespondierend dazu einen pumpengehäusefesten Einlassstutzen 417a, dessen Außenumfang an den Innenumfang der Ventillippe 418a angepasst ist.

Die Funktionsweise dieser Pumpe 414 ist die folgende: Bei einer Hubbewegung des Betätigungsgliedes 488 in Richtung 1a werden zunächst auch der Kolben 415 und der Ventilkörper 418 verlagert. Sobald dabei die Ventillippe 418a in Kontakt mit dem Einlassstutzen 417a gelangt und auf diesen aufgeschoben wird, ist die Pumpkammer 415 in Richtung eines Einlasskanals 414a abgeschlossen. Ein Weiterbewegen des Kolbens 416 führt dann zu einer druckbedingten Verlagerung des Ventilkörpers 418 in Richtung 1a, wobei diese Bewegung schneller als die Verlagerung des Kolbens 416 in gleiche Richtung abläuft. Daraus resultierend öffnet ein Pumpenauslassventil 419, welches durch eine konische Spitze 418b des Ventilkörpers 418 und einen Auslasskanal 416a im Kolben 416 gebildet ist. Der Austragvorgang beginnt. Wenn nachfolgend die manuelle Kraftbeaufschlagung des Betätigungsgliedes 488 entfällt, werden der Kolben 416 und der Ventilkörper 418 durch die Pumpenfeder in Rückhubrichtung 1b verlagert, wobei dies zunächst keine Neubefüllung der Pumpkammer 415 bewirkt, da die Ventillippe 418a für einen großen Teil des Rückhubweges noch am Einlassstutzen 417a anliegt. Stattdessen wird ein Unterdruck in der Pumpkammer 415 erzeugt. Erst wenn sich die Ventillippe 418a vom Einlassstutzen 417a löst und damit das Einlassventil 417 öffnet, führt dieser Unterdruck zu einem schlagartigen Einsaugen von Medium durch den Einlasskanal 414a. Diese Trennung von Ventil 418a und Einlassstutzen 417a findet erst gegen Ende des Rückhubs statt, sobald das Betätigungsglied 488 und das Sperrglied 452 außer Eingriff gelangen.

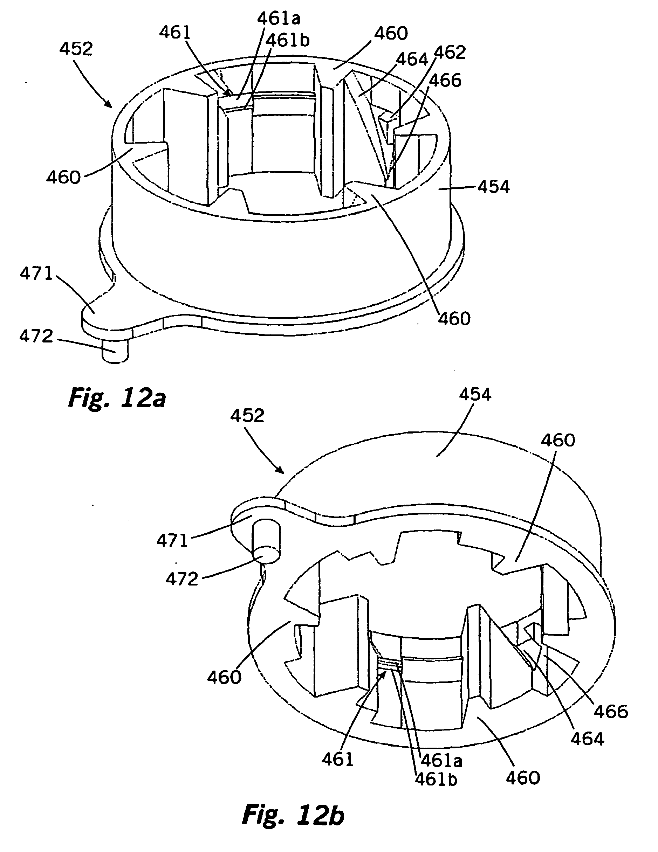

Die Verwendung einer solchen Pumpe 414, die über einen erheblichen Teil des Rückhubs die Pumpkammer 415 nicht neu befüllt und dies erst ab Erreichen einer Befüllungszwischenstellung des Kolbens tut, ergibt sich im Zusammenhang mit den Darstellungen der Fig. 12a und 12b. Diese Figuren zeigen in verschiedener Ansicht den Sperrring 452 der Austragvorrichtung gemäß der Fig. 11a. Dieser Sperrring 452 entspricht bezüglich der meisten Merkmale dem Sperrring, der in den Fig. 1e und 1f dargestellt ist. Er weist eine Mantelwandung 454 auf, von der aus sich radial nach außen ein Fortsatz 471 erstreckt, an dem ein Führungsstift 472 vorgesehen ist, der hinsichtlich seines Zwecks mit der Führungsgabel 72 der Ausführungsform der Fig. 1e und 1f übereinstimmt. Abweichend von der Ausgestaltung der Fig. 1e und 1f sind nicht insgesamt sechs Funktionsabschnitte, sondern stattdessen nur drei Funktionsabschnitte 460 mit Kulissenspurabschnitten 464, 466 an der Innenseite der Mantelwandung 454 angeformt. Zwischen diesen drei Funktionsabschnitten 460 sind jeweils Sperrstufen 461 angeordnet, die an ihrer nach oben gewandten Seite eine Schräge 461 a aufweisen und die an ihrer Unterseite eine im Wesentlichen radial ausgerichtete Sperrkante 461b aufweisen.

Die Wirkung dieser Sperrstufen 461 ist die folgende: Wenn das Betätigungsglied 488 aus einer nicht gesperrten Ausgangslage gemäß Fig. 11a in Richtung des Pfeils 1a hinabgedrückt wird, führt dies in der zu den vorangegangenen Ausführungsformen beschriebenen Art und Weise zunächst zu einem Verdrehen des Sperrrings 452 und gleichzeitig zum Beginn eines Medienaustrags. Sobald die Nocken 494 des Betätigungsgliedes 488 in den Bereich der Sperrstufen 461 gelangen, werden sie durch die Schrägen 461a radial nach innen ausgelenkt und springen nach Überwindung der Sperrstufen 461 im Bereich der Sperrkanten 461 b zurück in ihren unausgelenkten Zustand. Wenn dieser Zustand erreicht ist, ist eine Rückführung des Betätigungsgliedes 488 in die zuvor eingenommene Ausgangsstellung zunächst nicht mehr möglich, da die Nocken 494 nicht in Rückhubrichtung 1b über die Rückführstufen 461 hinübergeführt werden können, wie aus Fig. 11b ersichtlich ist. Demzufolge besteht die einzige Möglichkeit zur Rücküberführung des Betätigungsglieds 88 in seine unbetätigte Ausgangslage darin, dass die begonnene Hubbewegung in Richtung 1a zu Ende geführt wird, so dass nach Erreichen der Endlage gemäß Fig. 11c in der oben bereits beschriebenen Art und Weise eine Rückführung der Nocken 494 entlang der zweiten Kulissenspurabschnitte 466 erfolgt. Dies bringt allerdings in oben beschriebener Art zwingend eine Wiederherstellung des Sperrzustandes mit sich.

Diese Gestaltung, die nach Erreichen der Zwischenlage der Fig. 11b eine Rücküberführung in den Ausgangszustand der Fig. 11 a nur über Durchlaufen der Endstellung gemäß 11 c gestattet, verhindert in Verbindung mit der oben beschriebenen Gestaltung der Pumpe 414, dass ein Benutzer die Austragvorrichtung missbräuchlich verwendet, indem er wiederholte Teilhubbewegungen durchführt, bei denen die Nocken 494 zwischen der Sperrkante 461 b und der Unterkante 452a des Sperrgliedes 452 hin- und herbewegt werden. Eine solche Betätigung führt nicht zum Erfolg, da hierbei aufgrund der Gestaltung des Einlassventils 417 der Pumpe 414 keine Wiederbefüllung der Pumpkammer 415 erzielt werden kann. Wie oben bereits erwähnt wurde, wird die Pumpkammer 415 erst wieder dann befüllt, wenn die Rückhubbewegung in Richtung 1 b fast abgeschlossen oder abgeschlossen ist. Die Bewegung des Betätigungsgliedes 488 in Richtung 1 b, bis die Nocken 494 an den Sperrkanten 461b anschlagen, reicht hierfür nicht aus. Erst wenn die Nocken entlang der zweiten Kulissenspurabschnitten 466 in Richtung 1b gegenüber dem Sperrglied 452 verfahren werden, wird diese Neubefüllung der Pumpkammer 415 erzielt. Da hierdurch jedoch auch zwingend der Sperrzustand hergestellt wird, ist ein Missbrauch nicht möglich.

Bei einer nicht dargestellten Variante zur Ausführungsform der Fig. 11a bis 12b sind statt der jeweils nur einen Sperrstufe 461 je Nocke 494 eine Vielzahl von solchen Stufen hintereinander entlang der Hubbewegung 1a vorgesehen, so dass ein Rückhub fast vollständig verhindert wird, da im Zuge der Hubbewegung diese eine Rastleiter bildenden Sperrstufen jeweils nach Überwindung in Hubrichtung 1a eine Rückstellung in Rückhubrichtung 1 b verhindern.

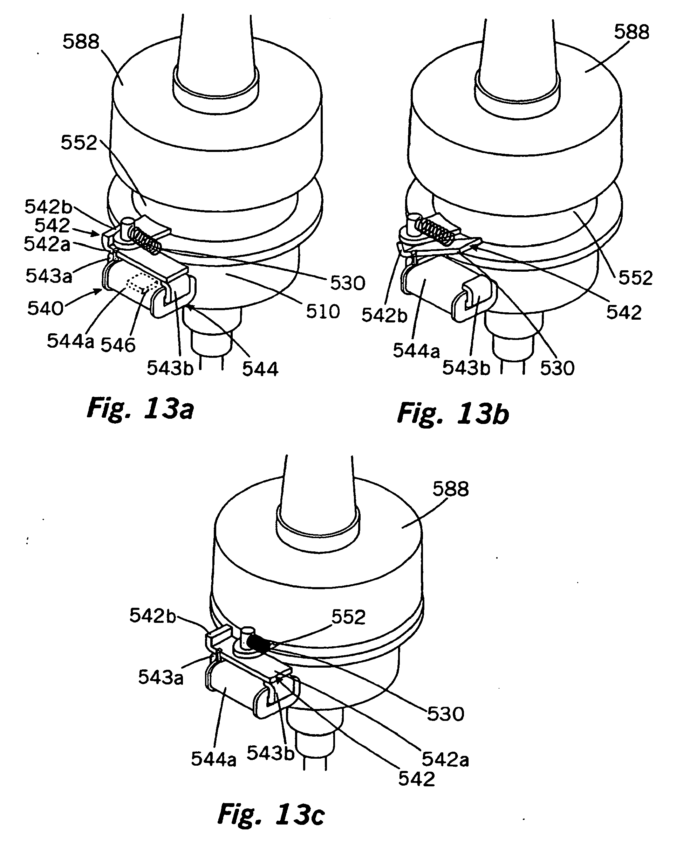

Die Fig. 13a bis 13c und zeigen eine weitere Ausführungsform einer erfindungsgemäßen Austragvorrichtung, die einen besonders einfachen Aufbau aufweist. Die Austragvorrichtung stimmt bezüglich der Gestaltung des Betätigungsgliedes 588 und des Sperrgliedes 552 weitgehend mit den Ausführungsformen der Fig. 1 bis 5 und 6 bis 8 überein.

Wie auch bei den genannten Ausführungsformen der Fig. 1 bis 8 ist bei dieser besonders einfachen Ausführungsform vorgesehen, dass das Sperrglied 552 durch eine Feder 532 permanent in Richtung der Freigabestellung, also bezogen auf die Perspektive der Figuren nach links, kraftbeaufschlagt wird. Die Feder 532 stützt sich hierfür in nicht näher dargestellter Weise rechtsseitig am Gehäuse 510 ab.

Im Sperrzustand der Fig. 13a wird das Sperrglied 552 durch eine Steueranordnung 540 davon abgehalten, durch die Feder 532 in die Freigabestellung der Fig. 13b verlagert zu werden. Diese Steueranordnung 540 umfasst ein Riegelglied 542, das mit einem langen Hauptabschnitt 542a und einem kurzen Blockierabschnitt 542b in etwa die Form eines liegenden "J" aufweist. Dieses Riegelglied 542, das beispielsweise als Metallteil oder als Metall-Kunststoff-Verbundteil ausgebildet sein kann, liegt in dem Blockierzustand der Fig. 13b mit zwei Endbereichen des Hauptabschnitts 542a jeweils auf Klammerabschnitten 543a, 543b aus einem Metall mit geringem magnetischen Widerstand, beispielsweise aus Eisen. Die Klammerabschnitten 543a, 543b liegen ihrerseits mit ihren nicht dargestellten und aufeinander zu weisenden inneren Enden an einem gestrichelt dargestellten Permanentmagneten 546 an. Im Blockierzustand der Fig. 13a ist der Hauptabschnitt 542a des Riegelgliedes 542 somit Teil einer an den beiden Polen des Permanentmagneten 546 anliegenden Feldführungseinrichtung. Hierdurch wird erreicht, dass trotz der vergleichsweise geringen Stärke des Permanentmagneten 546 die Federkraft der Feder 532 alleine nicht ausreicht, um das Sperrglied 552 unter Verlagerung des Blockierabschnitts 542b nach links in die Freigabestellung zu bewegen.

Zur Steueranordnung 540 gehört weiterhin auch eine Elektromagneteinrichtung 544 mit einer Spule 544a. Diese Spule 544a umgibt die innenseitigen Enden der Klammerabschnitte 543a, 543b sowie den Permanentmagneten 546. Durch Bestromung kann mittels dieser Spule 544a ein dem Magnetfeld des Permanentmagneten 546 entgegenwirkendes Magnetfeld erzeugt werden.

Zur Überführung der Austragvorrichtung in ihren Freigabezustand der Fig. 13b wird die Spule 544a für einen kurzen Augenblick bestromt. Dies führt dazu, dass das Magnetfeld des Permanentmagneten 546 derart geschwächt wird, dass die durch Feder 532 auf das Sperrglied 552 wirkende Kraft nach links ausreicht, um das Riegelglied 542 unter Ablösung dessen rechten Endbereichs von der dem rechtsseitigen Klammerabschnitt 543b in der in Fig. 13b gezeigten Art zu verschwenken. Durch diese Schwenkbewegung verlagert sich auch der Blockierabschnitt 542b, so dass das Sperrglied 552 freikommt und durch die Kraft der Feder 532 bis in die Freigabestellung der Fig. 13b verdreht wird. Die Austragvorrichtung befindet sich nun in ihrem Freigabezustand. In diesem Freigabezustand ist eine Bewegung des Betätigungsgliedes 588 nach unten möglich und dadurch ein Austragvorgang bewirkbar.

Wie unter anderem zu den Ausführungsformen der Fig. 1 bis 5 und 6 bis 8 bereits beschrieben, führt diese nun mögliche Betätigung des Betätigungsgliedes 588 zu einer Drehbewegung des Sperrgliedes 552 nach rechts, bei der vorliegenden Ausführungsform bis in die Stellung der Fig. 13c. Durch diese Bewegung des Sperrgliedes 552 wird der Hauptabschnitt 542a des Riegelgliedes 542 und damit auch der Blockierabschnitt 542b wieder in die Ausgangsstellung der Fig. 13a gedrückt. Der rechtsseitige Endbereich des Hauptabschnitts 542a des Riegelgliedes 542 kommt dadurch wieder in Kontakt mit dem rechtseitigen Klammerabschnitt 543b, so dass die Feldführungseinrichtung wieder geschlossen ist und das Riegelglied 542 aufgrund der inzwischen entfallenen Bestromung der Spule 544a mittelbar bewirkt durch den Permanentmagneten 546 in dieser Blockierstellung verbleibt. Mit dem Ende der Betätigung des Betätigungsgliedes 588 kommt aufgrund der Federkraft der Feder 532 auch das Sperrglied 552 wieder in Anlage mit dem Blockierabschnitt 542b des Riegelgliedes 542, so dass der Zustand der Fig. 13a wiederhergestellt ist, bis durch ein nicht dargestelltes Steuergerät die Spule 544a erneut bestromt wird.

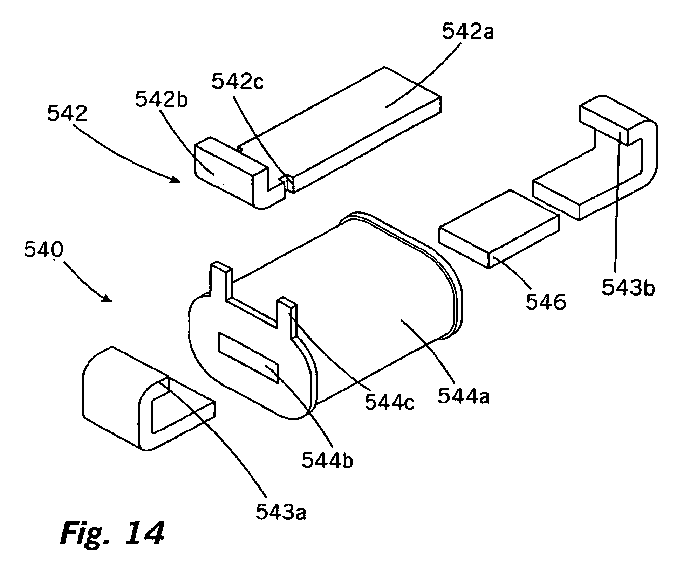

Fig. 14 verdeutlicht anhand einer Explosionsdarstellung noch einmal der Aufbau der Steueranordnung 540. Kern der Steueranordnung ist der Permanentmagnet 546, der in den durch die Spule 544a umgebenen Spuleninnenbereich 544b eingeschoben ist. Beidseitig sind weiterhin die Klammerabschnitte 543a, 543b teilweise in diesen Spuleninnenbereich 544b eingeschoben. Das Riegelglied 542 liegt im Blockierzustand, dargestellt in Fig. 13a, auf den gegenüberliegenden äußeren Enden der Klammerabschnitte 543a, 543b auf. Die Relativbeweglichkeit des Riegelgliedes 542 gegenüber den übrigen Komponenten der Steueranordnung 540 ist durch Ausnehmungen 542c im Riegelglied 542 und darin hineinragende Fortsätze 544c an der Elektromagneteinrichtung 544 auf die beschriebene Schwenkbewegung beschränkt, bei der das linkseitige Ende des Riegelgliedes 542 auf der gegenüberliegenden Außenseite des linken Klammerabschnitts 543a abrollt.

Bei nicht dargestellten Varianten dieser Ausführungsform der Fig. 13a bis 13c und 14 kann das Riegelglied auch durch eine Scharnieranordnung um eine genau definierte Schwenkachse gegenüber den übrigen Bauteilen der Steueranordnung verschwenkbar ausgebildet sein.

## Patentansprüche

1. Austragvorrichtung für Medien mit
- einem Gehäuse (10, 510),
- einem gegenüber dem Gehäuse (10, 510) manuell bewegbaren Betätigungsglied (88; 188; 288; 488; 588), das zum Zwecke der Betätigung eines Austragmittels (414) aus einer unbetätigten Ausgangslage in Richtung einer Betätigungsrichtung (1a) in eine betätigte Endlage überführbar ist, und
- einem Sperrglied (52; 152; 252; 452; 552), welches zwischen einer Sperrstellung, in der es die Verlagerung des Betätigungsgliedes (88; 188; 288; 488; 588) in die Endlage verhindert, und einer Freigabestellung, in der es die Verlagerung des Betätigungsgliedes (88; 188; 288; 488; 588) in die Endlage ermöglicht, gegenüber dem Gehäuse (10; 510) verlagerbar ist,
**dadurch gekennzeichnet, dass**
- ein Federmittel (32; 132; 232; 332; 532) vorgesehen ist, durch welches das Sperrglied (52; 152; 252; 452; 552) in Richtung seiner Freigabestellung kraftbeaufschlagt wird und
- das Betätigungsglied (88; 188; 288; 488; 588) und das Sperrglied (52; 152; 252; 452; 552) derart miteinander wirkgekoppelt sind, dass in der Freigabestellung eine Hubbewegung des Betätigungsgliedes (88; 188; 288; 488; 588) aus der unbetätigten Ausgangslage in die betätigte Endlage und/oder eine nachfolgende Rückhubbewegung aus der betätigten Endlage in die Ausgangslage mittels einer Übertragungsmechanik (64, 66, 94; 464, 466, 494) eine Energieeinspeisung in das erste Federmittel (32; 132; 232; 332; 532) bewirkt.

2. Austragvorrichtung nach Anspruch 1,
**dadurch gekennzeichnet, dass**
das Betätigungsglied (88; 188; 288; 488; 588) und das Sperrglied (52; 152; 252; 452; 552) über eine Kulissenführung (94, 64, 66; 464, 466, 494) miteinander gekoppelt sind, mittels derer die Hubbewegung und/oder die Rückhubbewegung des Betätigungsgliedes (88; 188; 288; 488; 588) die Verlagerung des Sperrgliedes (52; 152; 252; 452; 552) bewirkt.

3. Austragvorrichtung nach Anspruch 2,
**dadurch gekennzeichnet, dass**
die Kulissenführung (94, 64, 66; 464, 466, 494) mindestens eine Kulissenspur (64, 66; 464, 466) am Sperrglied (52; 152; 252; 452; 552) und mindestens eine Nocke (94; 492) zum Eingriff in die Kulissenspur (64, 66; 464, 466) am Betätigungsglied (88; 188; 288; 488; 588) aufweist, wobei die Kulissenführung (94, 64, 66; 464, 466, 494) derart ausgebildet ist, dass sie einen ersten Kulissenspurabschnitt (64; 464) aufweist, in den die Nocke (94; 494) bei der Hubbewegung einfährt, und/oder einen zweiten Kulissenspurabschnitt (66; 466) aufweist, in den die Nocke (94; 494) gegen Ende der Hubbewegung oder beim Übergang in die Rückhubbewegung einfährt.

4. Austragvorrichtung nach einem der vorstehenden Ansprüche,
**dadurch gekennzeichnet, dass**
eine Hubbewegung des Betätigungsgliedes (88; 188; 288; 488; 588) in der Sperrstellung des Sperrgliedes (52; 152; 252; 452; 552) **dadurch** zumindest abschnittsweise unterbunden wird, dass mindestens ein sperrgliedseitiger Sperrabschnitt (62; 462) den Hubbewegungspfad von mindestens einem betätigungsgliedseitigen Sperrabschnitt (94; 494) versperrt, wobei der betätigungsgliedseitige Sperrabschnitt (94; 494) vorzugsweise mit der der betätigungsgliedseitigen Nocke (94; 494) der Kulissenführung (94, 64, 66; 494, 464, 466) identisch ist.

5. Austragvorrichtung nach einem der vorstehenden Ansprüche
**dadurch gekennzeichnet, dass**
eine Steueranordnung (20; 120; 240; 540) vorgesehen ist, die dafür ausgebildet ist,
- in einem Blockierzustand die Bewegung des Sperrgliedes (52; 152; 252; 452; 552) gegenüber dem Gehäuse zu blockieren und
- durch eine mittels eines elektrisches Signal erzielbare Auslösung die Verlagerung des Sperrgliedes (52; 152; 252; 452; 552) aus der Sperrstellung in die Freigabestellung zu ermöglichen.

6. Austragvorrichtung nach Anspruch 5,
**dadurch gekennzeichnet, dass**
die Steueranordnung derart ausgebildet ist, dass sie bei einer Verlagerung des Sperrgliedes (52; 152; 252; 452; 552) in die Sperrstellung automatisch in den Blockierzustand überführt wird.

7. Austragvorrichtung nach Anspruch 5 oder 6,
**dadurch gekennzeichnet, dass**
ein Riegelglied (142; 242; 342; 542) vorgesehen ist, welches im Blockierzustand der Steueranordnung eine Verlagerung des Sperrgliedes (152; 252; 452; 552) oder eines mit dem Sperrglied wirkverbundenen Sperrhilfsgliedes (178; 278; 378) mechanisch blockiert.

8. Austragvorrichtung nach einem der Ansprüche 5 bis 7,
**dadurch gekennzeichnet, dass**
im Blockierzustand der Steueranordnung
- das Sperrglied oder ein mit dem Sperrglied (52; 452) wirkverbundenes Sperrhilfsglied (78) durch einen Permanentmagneten (42) in einer Lage gehalten wird, aus der resultierend das Sperrglied (52; 452) in seiner Sperrstellung angeordnet ist, oder
- das Riegelglied (542) oder ein mit dem Riegelglied wirkverbundenes Riegelhilfsglied (144; 244) durch einen Permanentmagneten (146; 246; 546) in einer Lage gehalten wird, aus der die mechanische Blockierung des Sperrgliedes (152; 252; 452; 552) durch das Riegelglied (142; 242; 542) resultiert.

9. Austragvorrichtung nach Anspruch 8,
**dadurch gekennzeichnet, dass**
die durch den Permanentmagneten (42; 146; 246; 446) in der Sperrstellung des Sperrgliedes (52; 152; 252; 452; 552) auf das Sperrglied (52; 152; 252; 452), das Sperrhilfsglied (78; 178; 278), das Riegelglied (142; 242; 542) und/oder das Riegelhitfsglied (144; 244) ausgeübte Kraft größer ist als die von Federmitteln (30, 32; 132; 232; 532) der Austragvorrichtung in der Sperrstellung des Sperrgliedes in entgegengesetzte Richtung auf das Sperrglied, das Sperrhilfsglied, das Riegelglied bzw. das Riegelhilfsglied wirkende Kraft.

10. Austragvorrichtung nach einem der Ansprüche 5 bis 9,
**dadurch gekennzeichnet, dass**
zur Erzielung der Auslösung ein elektrisch ansteuerbarer Aktuator (44; 147; 247; 544a), vorzugsweise ein Elektromagnet vorgesehen ist, durch den
- das Sperrglied oder ein mit dem Sperrglied wirkverbundenes Sperrhilfsglied (78) kraftbeaufschlagt werden kann, so dass resultierend das Sperrglied (52) in Richtung seiner Freigabestellung verlagert wird, oder
- ein Riegelglied (542) oder ein mit dem Riegelglied wirkverbundenes Riegelhilfsglied (144; 244) in eine Richtung (5b) kraftbeaufschlagt wird, die zu einem Lösen der mechanischen Blockierung des Sperrgliedes (152; 252; 452; 552) durch das Riegelglied (142; 242; 542) und damit zu einer Verlagerung des Sperrgliedes (152; 252; 452; 552) in Richtung seiner Freigabestellung führt.

11. Austragvorrichtung nach einem der Ansprüche 8 bis 10,
**dadurch gekennzeichnet, dass**
das Riegelglied (542) Teil einer Feldführungseinrichtung ist, die im Blockierzustand der Steueranordnung die Pole des Permanentmagneten (546) miteinander verbinden.

12. Austragvorrichtung nach Anspruch 10 oder 11,
**dadurch gekennzeichnet, dass**
der elektrisch ansteuerbare Aktuator (44; 147; 247; 544a) dafür ausgebildet ist, eine Kraft auf das Sperrglied, das Sperrhilfsglied (78), das Riegelglied (542) oder das Riegelhilfsglied (144; 244) auszuüben, die größer ist als die in entgegengesetzte Richtung (3a; 5a) wirkende resultierende Kraft aus der auf das entsprechende Glied wirkende Kraft des Permanentmagneten (42; 146; 246; 546) und vorzugsweise der Kraft eines das Glied kraftbeaufschlagenden Federmittels (30, 32; 132; 232; 532).

13. Austragvorrichtung nach einem der vorstehenden Ansprüche ,
**dadurch gekennzeichnet, dass**
das Sperrglied (52; 152; 252; 452; 552) zur Überführung aus der Sperrstellung in die Freigabestellung um eine in Betätigungsrichtung (1a) ausgerichtete Drehachse (1) drehbar ausgebildet ist und über seinen Umfang verteilt mindestens drei Sperrabschnitte (62; 462) aufweist, die in der Sperrstellung des Sperrgliedes (52; 152; 252; 452; 552) derart angeordnet sind, dass eine Hubbewegung des Betätigungsgliedes (88; 188; 288; 488; 588) verhindert wird.

14. Austragvorrichtung nach Anspruch 13,
**dadurch gekennzeichnet, dass**
das Sperrglied (52; 152; 252; 452; 552) das Betätigungsglied (88; 188; 288; 488; 588) umlaufend umgibt und die Drehachse (1) des Sperrgliedes (52; 152; 252; 452; 552) parallel zur Betätigungsrichtung (1 a) und vorzugsweise koaxial zu einer Mittelachse (1) der Austragvorrichtung ausgerichtet ist.

15. Austragvorrichtung nach einem der vorstehenden Ansprüche,
**dadurch gekennzeichnet, dass**
ein Piezomotor oder ein Elektromotor zur Bewegung des Sperrgliedes, der Sperrhilfsgliedes, des Riegelgliedes (342) oder des Riegelhilfsgliedes vorgesehen ist, wobei vorzugsweise der Motor zur Bewegung eines Nockengliedes (344), insbesondere vorzugsweise zur rotativen Bewegung einer Nockenscheibe (344), vorgesehen ist, wobei das Nockenglied (344) dafür ausgebildet ist,
- unmittelbar die Bewegung des Sperrgliedes oder eines Sperrhilfsgliedes (378) in der Sperrstellung des Sperrgliedes zu verhindern oder
- mittelbar über ein Riegelglied die Bewegung des Sperrgliedes oder eines Sperrhilfsgliedes in der Sperrstellung des Sperrgliedes zu verhindern, wobei vorzugsweise das Riegelglied in Richtung einer Auslösestellung federkraftbeaufschlagt ist und durch das Nockenglied in Abhängigkeit der Stellung des Nockengliedes in der Blockierstellung gehalten wird.

16. Austragvorrichtung nach einem der vorstehenden Ansprüche ,
**dadurch gekennzeichnet, dass**
eine Rückführsperre (461) vorgesehen ist, die nach Erreichen einer definierten Sperrzwischenstellung bei einer Überführung des Betätigungsgliedes (488) aus der Ausgangslage in Richtung der Endlage eine Rückführung des Betätigungsgliedes (488) in die Ausgangslage solange verhindert, bis das Betätigüngsglied (488) in die Endlage überführt wird.

17. Austragvorrichtung nach Anspruch 16,
**dadurch gekennzeichnet, dass**
das Austragmittel (414) eine Pumpe (414) mit einer volumenveränderlichen Pumpkammer (415) aufweist, wobei die Pumpe (414) derart gestaltet ist, dass eine Befüllung der Pumpkammer (415) im Rahmen eines Rückhubs erst ab einer definierten Befüllungszwischenstellung erfolgt, wobei diese Befüllungszwischenstellung derart angeordnet ist, dass sie dann erreicht wird, wenn sich das Betätigungsglied (488) in einer Lage zwischen der Sperrzwischenstellung und der Ausgangslage befindet.

18. Austragvorrichtung nach einem der Ansprüche 16 oder 17,
**dadurch gekennzeichnet, dass**
die Rückführsperre (461) Rastmittel (461b, 494) aufweist, wobei ein erster Bestandteil (461 b) der Rastmittel (461 b, 494) in Betätigungsrichtung (1 a) ortsfest zum Gehäuse angeordnet ist und wobei ein zweiter Bestandteil (494) der Rastmittel, der zum Zusammenwirken mit dem ersten Bestandteil (461 b) ausgebildet ist, in Betätigungsrichtung (1a) ortsfest zum Betätigungsglied (488) angeordnet ist.

## Claims

1. A discharge device for media, having
- a housing (10, 510),
- an actuating member (88; 188; 288; 488; 588) manually movable relative to the housing (10, 510), which may for the purpose of actuating a discharge means (414) be transferred from an unactuated starting position in an actuation direction (1a) into an actuated end position and
- a detent member (52; 152; 252; 452; 552), which may be displaced relative to the housing (10; 510) between a detent position, in which it prevents displacement of the actuating member (88; 188; 288; 488; 588) into the end position, and a release position, in which it allows displacement of the actuating member (88; 188; 288; 488; 588) into the end position, **characterised in that**
- a spring means (32; 132; 232; 332; 532) is provided, by which the detent member (52; 152; 252; 452; 552) is force-loaded towards its release position and
- the actuating member (88; 188; 288; 488; 588) and the detent member (52; 152; 252; 452; 552) are operatively coupled together in such a way that in the release position a travel movement of the actuating member (88; 188; 288; 488; 588) from the unactuated starting position into the actuated end position and/or a subsequent return travel movement from the actuated end position into the starting position feeds energy into the first spring means (32; 132; 232; 332; 532) by means of a transmission mechanism (64, 66, 94; 464, 466, 494).

2. A discharge device according to claim 1,
**characterised in that**
the actuating member (88; 188; 288; 488; 588) and the detent member (52; 152; 252; 452; 552) are coupled together via a slide guide (94, 64, 66; 464, 466, 494), by means of which the travel movement and/or the return travel movement of the actuating member (88; 188; 288; 488; 588) effects displacement of the detent member (52; 152; 252; 452; 552).

3. A discharge device according to claim 2,
**characterised in that**
the slide guide (94, 64, 66; 464, 466, 494) comprises at least one slide track (64, 66; 464, 466) on the detent member (52; 152; 252; 452; 552) and at least one cam (94; 492) for engagement in the slide track (64, 66; 464, 466) on the actuating member (88; 188; 288; 488; 588), the slide guide (94, 64, 66; 464, 466, 494) being configured such that it comprises a first slide track portion (64; 464), into which the cam (94; 494) travels during the travel movement, and/or a second slide track portion (66; 466), into which the cam (94; 494) travels towards the end of the travel movement or on transition to the return travel movement.

4. A discharge device according to any one of the preceding claims,
**characterised in that**
a travel movement of the actuating member (88; 188; 288; 488; 588) in the detent position of the detent member (52; 152; 252; 452; 552) is prevented at least in portions **in that** at least one detent portion (62; 462) on the detent member side blocks the travel movement path of at least one detent portion (94; 494) on the actuating member side, the detent portion (94; 494) on the actuating member side preferably being identical to the cam (94; 494), on the actuating member side, of the slide guide (94, 64, 66; 494, 464, 466).

5. A discharge device according to any one of the preceding claims,
**characterised in that**
a control arrangement (20; 120; 240; 540) is provided which is configured
- to block movement of the detent member (52; 152; 252; 452; 552) relative to the housing in a blocking state and
- to allow displacement of the detent member (52; 152; 252; 452; 552) out of the detent position into the release position by triggering achievable by means of an electrical signal.

6. A discharge device according to claim 5,
**characterised in that**
the control arrangement is configured such that it is transferred automatically into the blocking state on displacement of the detent member (52; 152; 252; 452; 552) into the detent position.

7. A discharge device according to claim 5 or claim 6,
**characterised in that**
a locking member (142; 242; 342; 542) is provided, which, when the control arrangement is in the blocking state, mechanically blocks displacement of the detent member (152; 252; 452; 552) or of an auxiliary detent member (178; 278; 378) operatively connected with the detent member.

8. A discharge device according to any one of claims 5 to 7,
**characterised in that**
when the control arrangement is in the blocking state
- the detent member or an auxiliary detent member (78) operatively connected to the detent member (52; 452) is held in a position by a permanent magnet (42), as a result of which the detent member (52; 452) is arranged in its detent position, or
- the locking member (542) or an auxiliary locking member (144; 244) operatively connected to the locking member is held by a permanent magnet (146; 246; 546) in a position which results in mechanical blocking of the detent member (152; 252; 452; 552) by the locking member (142; 242; 542).

9. A discharge device according to claim 8,
**characterised in that**
the force exerted by the permanent magnet (42; 146; 246; 446), when the detent member (52; 152; 252; 452; 552) is in the detent position, on the detent member (52; 152; 252; 452), the auxiliary detent member (78; 178; 278), the locking member (142; 242; 542) and/or the auxiliary locking member (144; 244) is greater than the force exerted by spring means (30, 32; 132; 232; 532) of the discharge device, when the detent member is in the detent position, in the opposite direction on the detent member, the auxiliary detent member, the locking member or the auxiliary locking member.

10. A discharge device according to any one of claims 5 to 9,
**characterised in that**
to achieve triggering, an electrically drivable actuator (44; 147; 247; 544a), preferably a solenoid, is provided, by means of which
- force may be exerted on the detent member or an auxiliary detent member (78) operatively connected to the detent member, such that as a result the detent member (52) is displaced towards its release position, or
- force is exerted on a locking member (542) or an auxiliary locking member (144; 244) operatively connected to the locking member in a direction (5b) which leads to release of the mechanical blocking of the detent member (152; 252; 452; 552) by the locking member (142; 242; 542) and thus to displacement of the detent member (152; 252; 452; 552) towards its release position.

11. A discharge device according to any one of claims 8 to 10,
**characterised in that**
the locking member (542) is part of a field guide device, which connects the poles of the permanent magnet (546) together when the control arrangement is in the blocking state.

12. A discharge device according to claim 10 or claim 11,
**characterised in that**
the electrically drivable actuator (44; 147; 247; 544a) is configured to exert a force on the detent member, the auxiliary detent member (78), the locking member (542) or the auxiliary locking member (144; 244) which is greater than the force acting in the opposite direction (3a; 5a) arising from the force of the permanent magnet (42; 146; 246; 546) acting on the corresponding member and preferably the force of a spring means (30, 32; 132; 232; 532) exerting force on the member.

13. A discharge device according to any one of the preceding claims,
**characterised in that**
for transfer out of the detent position into the release position, the detent member (52; 152; 252; 452; 552) is configured so as to be rotatable about an axis of rotation (1) oriented in the actuation direction (1a) and comprises at least three detent portions (62; 462) spread over its circumference, which are arranged in such a way in the detent position of the detent member (52; 152; 252; 452; 552) that a travel movement of the actuating member (88; 188; 288; 488; 588) is prevented.

14. A discharge device according to claim 13,
**characterised in that**
the detent member (52; 152; 252; 452; 552) circumferentially surrounds the actuating member (88; 188; 288; 488; 588) and the axis of rotation (1) of the detent member (52; 152; 252; 452; 552) is oriented parallel to the actuation direction (1 a) and preferably coaxially with a centre axis (1) of the discharge device.

15. A discharge device according to any one of the preceding claims,
**characterised in that**
a piezomotor or an electric motor for moving the detent member, the auxiliary detent member, the locking member (342) or the auxiliary locking member is provided, the motor preferably being provided to move a cam member (344), in particular preferably for rotary movement of a cam disc (344), the cam member (344) being configured
- to directly prevent movement of the detent member or of an auxiliary detent member (378) when the detent member is in the detent position or
- to prevent indirectly via a locking member movement of the detent member or of an auxiliary detent member when the detent member is in the detent position, the locking member preferably being acted on with spring force towards a triggering position and being held in the blocking position by the cam member as a function of the position of the cam member.

16. A discharge device according to any one of the preceding claims,
**characterised in that**
a return latch (461) is provided, which, once a defined intermediate detent position is reached during transfer of the actuating member (488) from the starting position towards the end position, prevents return of the actuating member (488) into the starting position until the actuating member (488) is transferred into the end position.

17. A discharge device according to claim 16,
**characterised in that**
the discharge means (414) comprises a pump (414) with a variable volume pump chamber (415), the pump (414) being configured such that filling of the pump chamber (415) takes place in the context of return travel only from a defined intermediate filling position, this intermediate filling position being arranged such that it is reached when the actuating member (488) is situated in a position between the intermediate detent position and the starting position.

18. A discharge device according to either one of claims 16 or 17,
**characterised in that**
the return latch (461) comprises latching means (461 b, 494), a first component (461 b) of the latching means (461 b, 494) being arranged stationarily relative to the housing in the actuation direction (1 a) and a second component (494) of the latching means, which is configured to cooperate with the first component (461b), being arranged stationarily relative to the actuating member (488) in the actuation direction (1 a).

## Revendications

1. Dispositif de distribution de fluides avec
- un carter (10, 510),
- un élément de commande (88 ; 188 ; 288 ; 488 ; 588) déplaçable manuellement par rapport au carter (10, 510), ledit élément de commande pouvant être déplacé, pour la commande d'un élément de distribution (414), d'une position initiale au repos dans le sens de la commande (1 a) jusqu'à une position finale actionnée et
- un élément de blocage (52 ; 152 ; 252 ; 452 ; 552), ce dernier pouvant être déplacé par rapport au carter (10 ; 510) entre une position de blocage, dans laquelle il empêche le déplacement de l'élément de commande (88 ; 188 ; 288 ; 488 ; 588) dans la position finale, et une position de déblocage, dans laquelle il permet le déplacement de l'élément de commande (88 ; 188 ; 288 ; 488 ; 588) dans la position finale,
**caractérisé en ce**
- **qu'**il est prévu un ressort (32 ; 132 ; 232 ; 332 ; 532), par le biais duquel l'élément de blocage (52 ; 152 ; 252 ; 452 ; 552) est sollicité par une force dans le sens de sa position de déblocage et
- l'élément de commande (88 ; 188 ; 288 ; 488 ; 588) et l'élément de blocage (52 ; 152 ; 252 ; 452 ; 552) sont raccordés ensemble de manière opérationnelle de sorte que, dans la position de déblocage, un mouvement de levage de l'élément de commande (88 ; 188 ; 288 ; 488 ; 588), de la position initiale au repos dans la position finale actionnée, et/ou un mouvement de recul consécutif, de la position finale actionnée dans la position initiale, engendre(nt) à l'aide d'un système mécanique de transmission (64, 66, 94 ; 464, 466, 494) une injection d'énergie dans le premier ressort (32 ; 132 ; 232 ; 332 ; 532).

2. Dispositif de distribution selon la revendication 1,
**caractérisé en ce**
**que** l'élément de commande (88 ; 188 ; 288 ; 488 ; 588) et l'élément de blocage (52 ; 152 ; 252 ; 452 ; 552) sont raccordés ensemble par l'intermédiaire d'un guide de coulisse (94, 64, 66 ; 464, 466, 494), au moyen duquel le mouvement de levage et/ou le mouvement de recul de l'élément de commande (88 ; 188 ; 288 ; 488 ; 588) provoque(nt) le déplacement de l'élément de blocage (52 ; 152 ; 252 ; 452 ; 552).

3. Dispositif de distribution selon la revendication 2,
**caractérisé en ce**
**que** le guide de coulisse (94, 64, 66 ; 464, 466, 494) comprend au moins une piste de coulisse (64, 66 ; 464, 466) sur l'élément de blocage (52; 152 ; 252 ; 452; 552) et au moins une came (94 ; 492) s'engrenant dans la piste de coulisse (64, 66 ; 464, 466) sur l'élément de commande (88 ; 188 ; 288 ; 488 ; 588), sachant que le guide de coulisse (94, 64, 66 ; 464, 466, 494) est conçu de sorte qu'il comprend une première section de piste de coulisse (64 ; 464), dans laquelle pénètre la came (94 ; 494) lors du mouvement de levage, et/ou comprend une seconde section de piste de coulisse (66 ; 466), dans laquelle pénètre la came (94; 494) vers la fin du mouvement de levage ou lors du passage dans le mouvement de recul.

4. Dispositif de distribution selon l'une des revendications précédentes,
**caractérisé en ce**
**qu'**un mouvement de levage de l'élément de commande (88 ; 188 ; 288 ; 488 ; 588) en position de blocage de l'élément de blocage (52 ; 152 ; 252 ; 452 ; 552) est empêché au moins partiellement par le fait qu'au moins une section de blocage (62 ; 462) du côté de l'élément de blocage bloque le trajet du mouvement de levage d'au moins une section de blocage (94 ; 494) du côté de l'élément de commande, sachant que la section de blocage (94 ; 494) du côté de l'élément de commande est de préférence identique à celle de la came (94 ; 494) - du côté de l'élément de commande - du guide de coulisse (94, 64, 66 ; 494, 464, 466).

5. Dispositif de distribution selon l'une des revendications précédentes,
**caractérisé en ce**
**qu'**il est prévu un dispositif de commande (20 ; 120 ; 240 ; 540), lequel est conçu pour,
- en état de blocage, bloquer le mouvement de l'élément de blocage (52 ; 152 ; 252 ; 452 ; 552) par rapport au carter et
- permettre, par un déclenchement obtenu au moyen d'un signal électrique, le déplacement de l'élément de blocage (52 ; 152 ; 252 ; 452 ; 552) de la position de blocage dans la position de déblocage.

6. Dispositif de distribution selon la revendication 5,
**caractérisé en ce**
**que** le dispositif de commande est conçu de sorte que, lors d'un déplacement de l'élément de blocage (52 ; 152 ; 252 ; 452 ; 552) en position de blocage, il est automatiquement placé en état de blocage.

7. Dispositif de distribution selon la revendication 5 ou 6,
**caractérisé en ce**
**qu'**il est prévu un élément de verrouillage (142 ; 242 ; 342 ; 542), lequel, en état de blocage du dispositif de commande, bloque mécaniquement un déplacement de l'élément de blocage (152 ; 252 ; 452 ; 552) ou d'un élément de blocage auxiliaire (178 ; 278 ; 378) en liaison active avec l'élément de blocage.

8. Dispositif de distribution selon l'une des revendications 5 à 7
**caractérisé en ce**
**que**, en état de blocage du dispositif de commande,
- l'élément de blocage ou un élément de blocage auxiliaire (78) en liaison active avec l'élément de blocage (52 ; 452) est maintenu par un aimant permanent (42) dans une position, ce qui a pour effet que l'élément de blocage (52 ; 452) est disposé dans sa position de blocage, ou
- l'élément de verrouillage (542) ou un élément de verrouillage auxiliaire (144 ; 244) en liaison active avec l'élément de verrouillage est maintenu par un aimant permanent (146 ; 246 ; 546) dans une position qui permet d'obtenir le blocage mécanique de l'élément de blocage (152 ; 252 ; 452 ; 552) par l'élément de verrouillage (142 ; 242 ; 542).

9. Dispositif de distribution selon la revendication 8,
**caractérisé en ce**
**que** la force exercée par l'aimant permanent (42 ; 146 ; 246 ; 446), en position de blocage de l'élément de blocage (52 ; 152 ; 252 ; 452 ; 552), sur l'élément de blocage (52 ; 152 ; 252 ; 452), l'élément de blocage auxiliaire (78 ; 178 ; 278), l'élément de verrouillage (142 ; 242 ; 542) et/ou l'élément de verrouillage auxiliaire (144 ; 244) est supérieure à la force des ressorts (30, 32 ; 132 ; 232 ; 532) du dispositif de distribution, en position de blocage de l'élément de blocage dans le sens opposé, agissant sur l'élément de blocage, l'élément de blocage auxiliaire, l'élément de verrouillage ou l'élément de verrouillage auxiliaire.

10. Dispositif de distribution selon l'une des revendications 5 à 9,
**caractérisé en ce**
**qu'**il est prévu un actionneur à commande électrique (44 ; 147; 247 ; 544a) pour l'obtention du déclenchement, de préférence un électroaimant, grâce auquel
- l'élément de blocage ou un élément de blocage auxiliaire (78) en liaison active avec l'élément de blocage peut être sollicité par une force, ce qui a pour effet que l'élément de blocage (52) est déplacé dans le sens de sa position de déblocage, ou
- un élément de verrouillage (542) ou un élément de verrouillage auxiliaire (144 ; 244) en liaison active avec l'élément de verrouillage est sollicité par une force dans une direction (5b), qui provoque un déblocage du blocage mécanique de l'élément de blocage (152 ; 252 ; 452 ; 552) par l'élément de verrouillage (142 ; 242 ; 542) et qui provoque par conséquent un déplacement de l'élément de blocage (152 ; 252 ; 452 ; 552) dans le sens de sa position de déblocage.

11. Dispositif de distribution selon l'une des revendications 8 à 10,
**caractérisé en ce**
**que** l'élément de verrouillage (542) fait partie d'un dispositif de guidage de champ qui, en état de blocage du dispositif de commande, relie les pôles de l'aimant permanent (546) entre eux.

12. Dispositif de distribution selon la revendication 10 ou 11,
**caractérisé en ce**
**que** l'actionneur à commande électrique (44 ; 147 ; 247 ; 544a) est conçu pour exercer sur l'élément de blocage, sur l'élément de blocage auxiliaire (78), sur l'élément de verrouillage (542) ou sur l'élément de verrouillage auxiliaire (144 ; 244), une force qui est supérieure à la force agissant dans la direction opposée (3a ; 5a) et résultant de la force de l'aimant permanent (42 ; 146 ; 246 ; 546) agissant sur l'élément correspondant, et supérieure à la force résultant de préférence de la force d'un ressort (30, 32 ; 132 ; 232 ; 532) sollicitant l'élément.

13. Dispositif de distribution selon l'une des revendications précédentes,
**caractérisé en ce**
**que**, pour le passage de la position de blocage dans la position de déblocage, l'élément de blocage (52 ; 152 ; 252 ; 452 ; 552) est conçu en étant rotatif autour d'un axe de rotation (1) aligné dans un sens d'actionnement (1 a) et comprend au moins trois sections de blocage (62 ; 462) réparties sur son périmètre qui, en position de blocage de l'élément de blocage (52 ; 152 ; 252 ; 452 ; 552), sont disposées de sorte qu'un mouvement de levage de l'élément de commande (88 ; 188 ; 288 ; 488 ; 588) est empêché.

14. Dispositif de distribution selon la revendication 13,
**caractérisé en ce**
**que** l'élément de blocage (52 ; 152 ; 252 ; 452 ; 552) entoure en périphérie l'élément de commande (88 ; 188 ; 288 ; 488 ; 588) et que l'axe de rotation (1) de l'élément de blocage (52 ; 152 ; 252 ; 452 ; 552) est orienté parallèlement au sens d'actionnement (1a) et de préférence coaxialement à un axe central (1) du dispositif de distribution.

15. Dispositif de distribution selon l'une des revendications précédentes,
**caractérisé en ce**
**qu'**il est prévu un moteur piézoélectrique ou un moteur électrique pour le mouvement de l'élément de blocage, de l'élément de blocage auxiliaire, de l'élément de verrouillage (342) ou de l'élément de verrouillage auxiliaire, sachant que le moteur est prévu de préférence pour le mouvement d'un élément de came (344), en particulier et de préférence pour un mouvement rotatif d'un élément de came (344), l'élément de came (344) étant conçu
- pour empêcher directement le mouvement de l'élément de blocage ou d'un élément de blocage auxiliaire (378) en position de blocage de l'élément de blocage ou
- pour empêcher indirectement, via un l'élément de verrouillage, le mouvement de l'élément de blocage ou d'un élément de blocage auxiliaire, en position de blocage de l'élément de blocage, sachant que de préférence l'élément de verrouillage est sollicité par une force de ressort dans le sens d'une position de déclenchement et est maintenu en position de blocage par l'élément de came en fonction de la position de l'élément de came.

16. Dispositif de distribution selon l'une des revendications précédentes,
**caractérisé en ce**
**qu'**il est prévu un dispositif de blocage anti-retour (461), lequel, lors de l'atteinte d'une position intermédiaire de blocage définie lors du passage de l'élément de commande (488) de la position initiale dans la direction de la position finale, empêche un réacheminement de l'élément de commande (488) dans la position initiale, jusqu'à ce que l'élément de commande (488) soit transféré dans la position finale.

17. Dispositif de distribution selon la revendication 16,
**caractérisé en ce que**
l'élément de distribution (414) comprend une pompe (414) avec une chambre de pompage à volume variable (415), sachant que la pompe (414) est conçue de sorte qu'un remplissage de la chambre de pompage (415) dans le cadre d'une course de retour n'est effectué qu'à partir d'une position intermédiaire de remplissage définie, sachant que cette position intermédiaire de remplissage est disposée de sorte qu'elle n'est atteinte que si l'élément de commande (488) se trouve entre la position intermédiaire de blocage et la position initiale.

18. Dispositif de distribution selon l'une des revendications 16 ou 17,
**caractérisé en ce que**
le dispositif de blocage anti-retour (461) présente des éléments d'arrêt (461 b, 494), sachant qu'un premier composant (461 b) des éléments d'arrêt (461 b, 494) est disposé de manière fixe dans le sens de la commande (1 a) par rapport au carter et sachant qu'un second composant (494) des éléments d'arrêt, qui est conçu pour une interaction avec le premier composant (461 b), est disposé de manière fixe dans le sens de la commande (1 a) par rapport à l'élément de commande (488).
